# EUROPÄISCHE PATENTSCHRIFT

(11) **EP 4 592 671 B1**
(45) Veröffentlichungstag und Bekanntmachung des Hinweises auf die Patenterteilung: **19.08.2026**
(21) Anmeldenummer: 25151426.1
(22) Anmeldetag: 13.01.2025
(51) Int. Cl.: G01N 25/48, G01N 33/44, G01K 17/00

(54) **VERFAHREN ZUM IDENTIFIZIEREN MINDESTENS EINER KOMPONENTE EINES PROBEMATERIALS**
METHOD FOR IDENTIFYING AT LEAST ONE COMPONENT OF A SAMPLE MATERIAL
PROCÉDÉ D'IDENTIFICATION D'AU MOINS UN COMPOSANT D'UN MATÉRIAU ÉCHANTILLON

(30) Priorität: 29.01.2024 DE 102024102474
(43) Veröffentlichungstag der Anmeldung: 30.07.2025
(73) Patentinhaber: Netzsch-Gerätebau GmbH, 95100 Selb (DE)
(72) Erfinder: Storch, Georg, 95100 Selb (DE); Rudolph, Natalie, 97776 Eußenheim (DE); Gschwendtner, Reinhard, 95615 Marktredwitz (DE); Beckstein, Fabian, 95032 Hof (DE)

(56) Entgegenhaltungen:
- WO-A1-2022/250533
- WO-A1-2023/083937
- DE-A1- 2 350 932

## Beschreibung

### GEBIET DER ERFINDUNG

Die Erfindung bezieht sich auf ein Verfahren zum Identifizieren mindestens einer Komponente eines Probematerials.

### ALLGEMEINER STAND DER TECHNIK

Wenngleich die Erfindung im Zusammenhang mit dem Identifizieren von Komponenten in Probematerialien unterschiedlichster Art und auf unterschiedlichsten Gebieten nützlich sein und Verwendung finden kann, sollen die Erfindung und die ihr zu Grunde liegende Problematik nachfolgend am Beispiel des Kunststoffrecyclings exemplarisch näher erläutert werden, ohne jedoch die Erfindung dahingehend einzuschränken.

Auf dem Gebiet des Recyclings von Kunststoffen stellt sich vielfach die Aufgabe, auf verschiedenen Stufen des Recyclingprozesses die Zusammensetzung des recyclierten Kunststoffmaterials, zum Beispiel eines Kunststoffgranulats, zu bestimmen und eine Aussage über die Reinheit dieses Materials sowie über das Ausmaß eventueller Beimischungen zu erhalten. Insbesondere sollen Anteile eventuell im untersuchten Material, insbesondere Recyclat, vorhandener, unerwünschter Polymersorten detektiert werden.

Verbreitete Verfahren auf dem Gebiet des Kunststoffrecyclings, die eher für den Vorgang des Sortierens des Ausgangsmaterials gedacht sind, sind die IR-Spektroskopie, die Nutzung von Kameras in Verbindung mit Bilderkennung, weitere optische Verfahren, oder eine Trennung der Materialien nach deren Dichte. Keine dieser herkömmlichen Methoden kann jedoch den gesamten Bereich relevanter Kunststoffe abdecken. So ist beispielsweise die IR-Spektroskopie auf schwarze Kunststoffe nicht sensibel. Insbesondere scheitern die vorgenannten Verfahren zudem bei der Analyse von Komponenten in compoundierten Stoffgemischen, etwa zum Zweck der Qualitätskontrolle.

Es ist bekannt, mittels des Verfahrens der so genannten dynamischen Differenzkalorimetrie (im Englischen als differential scanning calorimetry, abgekürzt DSC, bezeichnet) beispielsweise Schmelz- oder Glasübergangstemperaturen, insbesondere von Kunststoffen, zu bestimmen. Es ist hierbei möglich, anhand von Peaks der mittels des DSC-Verfahrens erhaltenen Meßgröße auf die Art des untersuchten Kunststoffs zu schließen. Das Verfahren der dynamischen Wärmestrom-Differenzkalorimetrie ist grundsätzlich in der Lage, viele Kunststoffe zu erkennen und bietet eine hohe Empfindlichkeit.

In der Kunststoffrecyclingbranche werden jedoch große Materialmengen verarbeitet, die auf deren Reinheit und Zusammensetzung hin überprüft werden sollen. Die in herkömmlicher Weise mittels dynamischer Differenzkalorimetrie und den hierfür verwendeten Vorrichtungen untersuchten Materialproben sind aber sehr klein, deren Mengen liegen im Bereich von Milligramm. Die Untersuchung einer einzelnen Probe aus einer großen Materialcharge, etwa aus einem mit Kunststoffgranulat gefüllten Silo oder ähnlichem, mittels DSC ermöglicht es folglich nicht, ein zuverlässiges, repräsentatives Bild der gesamten Charge zu gewinnen. Eine Verbesserung der Aussage durch Untersuchung einer Vielzahl von Proben aus der Charge mittels DSC ist sehr aufwändig und zeitraubend.

In dem Artikel "Simultane thermische Analyse an großen Proben" von G. Matuschek et al., Journal of Thermal Analysis, Vol. 47 (1996), S. 623ff. ist ein Gerät zur Durchführung einer STA (Simultanen Thermischen Analyse) für größere Einwaagen beschrieben. Dieses Instrument ist mit Blick auf einen Einsatz im Recyclingbereich jedoch in der Anschaffung relativ teuer und in der Anwendung relativ kompliziert. Zudem kann die Probenverteilung ungünstig sein. Für den Einsatz im Recyclingbereich wäre eine kostengünstigere, in der Handhabung einfachere Herangehensweise wünschenswert.

Ferner sind als HFM-Gerät bezeichnete Apparaturen bekannt, wobei HFM für den englischen Ausdruck "heat flow meter" steht. Mittels einer solchen Apparatur wird herkömmlicherweise der Wärmedurchgang durch eine Probe gemessen, zum Beispiel um die Isoliereigenschaften eines Isoliermaterials zu bestimmen. Hierzu wird die Probe in der HFM-Apparatur zwischen zwei temperierbaren Platten angeordnet, mit Hilfe der Platten ein Temperaturgradient auf die Probe aufgebracht und, im stationären Zustand nach Erreichen eines thermischen Gleichgewichts, der durch die Probe fließende Wärmestrom gemessen und damit der Wärmedurchgang zahlenmäßig bestimmt.

Ebenfalls ist es bekannt, die spezifische Wärmekapazität Cp beispielsweise eines Polymers oder eines Isoliermaterials mittels einer HFM-Apparatur zu messen. Hierzu werden beide Platten zunächst bei gleicher Temperatur gehalten und dann eine kurze Temperaturrampe gefahren, gefolgt erneut von isothermen Bedingungen. Das Integral des Wärmestroms wird hierbei ausgewertet.

Darüber hinaus ist im technischen Standard ASTM C1784 eine Betriebsweise einer HFM-Apparatur beschrieben, bei der beide Platten auf gleiche Temperatur gebracht werden, wobei ein stufenweise isothermes Temperaturprogramm der Bestimmung des Wärmespeichervermögens einer Probe, im Sinne sensibler und latenter Wärme, dient.

Die WO 2022/250533 A1 beschäftigt sich mit einem Verfahren der dynamischen Differenzkalorimetrie sowie einer Vorrichtung für eine dynamische Differenzkalorimetrie, wobei die Bestimmung thermodynamischer Eigenschaften eines Materials sowie der Zusammensetzung desselben beschrieben werden. Bei der in der WO 2022/250533 A1 beschriebenen Vorgehensweise wird die plattenförmige Probe auf einer Seite beheizt oder gekühlt. Auf der gegenüberliegenden Seite der Probe ist eine Vielzahl von in einem Raster flächig verteilt angeordneten Temperatursensoren vorgesehen.

Es wäre vor diesem Hintergrund wünschenswert, auf einfache, gut handhabbare sowie zeit- und kosteneffiziente Weise eine größere Charge eines Materials, beispielsweise auf dem Gebiet des Kunststoffrecyclings, zutreffend charakterisieren und hierbei ein genaues Bild über deren Zusammensetzung und eventuelle Verunreinigungen gewinnen zu können.

### KURZDARSTELLUNG DER ERFINDUNG

Vor diesem Hintergrund ist es eine Aufgabe der Erfindung, ein effizientes und zuverlässiges Verfahren anzugeben, mittels desselben eine ausreichend große Probemenge untersucht werden kann, um eine Materialcharge möglichst repräsentativ zu charakterisieren. Insbesondere soll das Verfahren zur zutreffenden Charakterisierung von Materialchargen in industriellen Größenordnungen mittels Probenahme und -untersuchung geeignet und zweckmäßig sein.

Erfindungsgemäß wird diese Aufgabe durch ein Verfahren mit den Merkmalen des Anspruchs 1 gelöst.

Demgemäß wird ein Verfahren zum Identifizieren mindestens einer Komponente eines Probematerials, mit dem eine Probe gebildet ist, vorgeschlagen, wobei das Verfahren umfasst:
- Bereitstellen der Probe, wobei das Probematerial mit einer platten- oder scheibenartigen Geometrie ausgebildet wird oder das Probematerial in einem Raumgebiet mit einer platten- oder scheibenartigen Geometrie angeordnet wird;
- Anordnen der Probe zwischen zwei temperierbaren plattenförmigen Elementen derart, dass sich die temperierbaren plattenförmigen Elemente entlang jeweils einer von zwei entgegengesetzten Hauptoberflächen der platten- oder scheibenartigen Geometrie erstrecken;
- Herbeiführen einer Temperaturänderung der Probe durch Herbeiführen eines Wärmezuflusses in die Probe oder eines Wärmeabflusses aus der Probe in einer symmetrischen Weise durch die beiden Hauptoberflächen der platten- oder scheibenartigen Geometrie, wobei die Temperaturänderung mittels Temperieren der plattenförmigen Elemente bewirkt wird und einen Temperaturbereich bestreicht, innerhalb dessen mindestens eine endotherme oder exotherme Veränderung der mindestens einen Komponente des Probematerials auftritt;
- Erfassen eines in die Probe hinein oder aus der Probe heraus fließenden Wärmestroms und hieraus Erhalten eines als Kurve darstellbaren Verlaufs des Wärmestroms in Abhängigkeit von einer Temperatur der Probe oder der temperierbaren plattenförmigen Elemente oder in Abhängigkeit von der Zeit, oder
   Erfassen einer Temperatur der Probe oder der temperierbaren plattenförmigen Elemente und hieraus Erhalten eines als Kurve darstellbaren Verlaufs einer zeitlichen Änderung der Temperatur in Abhängigkeit von der Temperatur oder in Abhängigkeit von der Zeit;
- Vergleichen einer Kurvenbeschaffenheit, insbesondere einschließlich einer Kurvenform, des erhaltenen Verlaufs des Wärmestroms oder der zeitlichen Änderung der Temperatur über der Temperatur oder der Zeit jeweils mit einer Referenzkurvenbeschaffenheit eines oder mehrerer Referenzverläufe; und
- Identifizieren der mindestens einen Komponente des Probematerials basierend auf dem Ergebnis des Vergleichs.

Eine der Erfindung zu Grunde liegende Idee besteht darin, das Probematerial grundsätzlich angelehnt an das Verfahren der dynamischen Differenzkalorimetrie (DSC) zu untersuchen, jedoch an einer Probe, die eine sehr viel größere Materialmenge als bei der DSC enthält. Auf diese Weise gelingt es, eine inhomogene Zusammensetzung des zu untersuchenden Materials, zum Beispiel Recyclats, durch die Probe in erheblich verbesserter Weise repräsentativ abzubilden und die Komponente(n) eines Gemisches zutreffend zu identifizieren.

Bei der Erfindung kann hierzu eine als solche bekannte HFM-Anordnung in einer DSC-ähnlichen Weise benutzt werden. Die HFM-Apparatur kann hierbei einer Apparatur, wie diese beispielsweise im technischen Standard ASTM C1784 und darin angeführten Referenzen genannt ist, entsprechen oder dieser ähnlich ausgebildet sein. Je nach den zu untersuchenden Probematerialien kann die HFM-Apparatur jedoch zum Beispiel hinsichtlich des Temperaturbereichs, der bestrichen werden kann, modifiziert sein. So kann es für die vorteilhafte Anwendung auf den Bereich des Kunststoffrecyclings zweckmäßig sein, wenn die HFM-Apparatur mittels modifizierter Temperiereinrichtungen einen größeren Temperaturbereich und höhere Maximaltemperaturen möglich macht.

Vorteilhaft können mit dem erfindungsgemäßen Verfahren durch das im Wesentlichen symmetrische Einbringen oder Abführen von Wärme durch die beiden Hauptoberflächen, mit anderen Worten die symmetrische Beaufschlagung der Probe mit einem Wärmestrom oder das symmetrische Einprägen einer Temperaturänderung, in Verbindung mit der platten- oder scheibenförmigen Geometrie des Probematerials bzw. des Gebiets, in dem dieses angeordnet wird, der Wärmeverlust nach außen hin vermindert sowie wohldefinierte Temperaturgradienten und insbesondere in einem Mittenbereich des Probematerials ein zumindest in sehr guter Näherung eindimensionaler Wärmestrom erzielt werden. Dies trägt vorteilhaft zu genauen, zuverlässigen Messergebnissen und einer zuverlässigen Identifikation der Komponente(n) bei.

Die platten- oder scheibenförmige Geometrie des Probematerials ist zudem dahingehend vorteilhaft, dass der durch die Wärmezufuhr oder -abfuhr induzierte endo-/exotherme Vorgang im Probematerial, etwa ein Schmelz- oder Glasübergang einer Komponente desselben, zügig erfolgt und sich somit keine wesentlichen Temperaturgradienten und damit auch keine "Schmelzfront" innerhalb der Probe aufbauen kann. Dies wird erreicht, indem durch die vorgesehene Geometrie trotz relativ großer Einwaage eine große Wärmeeintragsfläche ermöglicht wird.

Zudem gelingt bei der vorliegenden Erfindung vorteilhaft die Identifikation der Komponente(n), ohne dass eine gleichzeitige Messung an einem Referenzmaterial oder eine gleichzeitige Leermessung als Referenz erforderlich wird. Dies trägt zu einer Verringerung des apparativen Aufwands und der damit verbundenen Kosten sowie zu einer Vereinfachung bei der Durchführung des Verfahrens bei. Die Identifikation der Komponente(n) ist somit nicht nur zuverlässig, sondern auch zeitsparend und in einfacher Weise möglich und somit vorteilhaft für den Einsatz zum Beispiel im Bereich des Kunststoffrecyclings, oder anderer Prozesse, in einem industriellen Maßstab.

Vorteilhafte Ausgestaltungen und Weiterbildungen der Erfindung ergeben sich aus den Unteransprüchen sowie aus der Beschreibung unter Bezugnahme auf die Zeichnungen.

In einer Ausgestaltung wird bei dem Herbeiführen der Temperaturänderung der Probe das Probematerial einem kontinuierlichen Temperaturverlauf, insbesondere einer vordefinierten Temperaturrampe, vorzugsweise einer linear mit der Zeit ansteigenden oder abfallenden Temperaturrampe, unterworfen.

In einer Weiterbildung erfolgt das Herbeiführen der Temperaturänderung der Probe in der Weise, dass ein über der Zeit konstanter Wärmestrom der Probe zugeführt oder aus der Probe abgeführt wird.

Gemäß einer Ausgestaltung durchläuft innerhalb des bei der Temperaturänderung bestrichenen Temperaturbereichs die mindestens eine Komponente des Probematerials einen Phasenübergang und/oder einen Glasübergang. Derartige Veränderungen der Komponenten des Probematerials mit der Temperatur desselben können gut zur Identifikation einer oder mehrerer Komponenten herangezogen werden.

In einer Weiterbildung wird das Probematerial während der Temperaturänderung ganz oder teilweise aufgeschmolzen oder eingefroren. Schmelzen oder Gefrieren als Phasenübergänge zwischen fester und flüssiger Phase sind ebenfalls dienlich zur Charakterisierung der Komponente(n) des Probematerials.

Insbesondere dienen die temperierbaren plattenförmigen Elemente dem Heizen und/oder Kühlen der Probe.

Bei einer Ausgestaltung wird das Probematerial mittels Temperierens der temperierbaren plattenförmigen Elemente zwei oder mehr Temperaturänderungsvorgängen unterzogen, insbesondere einem oder mehreren Heizvorgängen und einem oder mehreren Kühlvorgängen. Dies kann zu einer noch besseren Identifizierung der Komponente(n) genutzt werden. Mit Hilfe definierter Aufheiz- und/oder Abkühlraten kann, je nach Probematerial bzw. dessen Komponenten, auf die temperaturabhängigen Vorgänge im Probematerial Einfluss genommen werden. Eine zweite Aufheizung, und deren Auswertung, kann insbesondere genutzt werden, um einen einheitlichen Ausgangszustand herzustellen, beispielsweise durch vorangehendes Aufschmelzen, und die Auswertung ausgehend von einem derartigen definierten Ausgangszustand durchzuführen. Insbesondere die zweite Aufheizung sowie ferner eine sich anschließende Abkühlphase, während der beispielsweise eine Kristallisation beobachtet werden kann, können für den Vergleich der Kurvenbeschaffenheit mit der Referenzkurvenbeschaffenheit interessant sein und herangezogen werden. Eine derartige Abkühlphase kann zum Beispiel Rückschlüsse auf eventuell in der Probe enthaltene Additive als Kristallisationsbeschleuniger ermöglichen.

Gemäß einer Ausgestaltung wird ein jeweils von dem temperierbaren plattenförmigen Element in die Probe oder umgekehrt fließender Wärmestrom jeweils zwischen dem temperierbaren plattenförmigen Element und der dem temperierbaren plattenförmigen Element zugewandten Seite der Probe im Bereich der Hauptoberfläche der platten- oder scheibenartigen Geometrie, der das temperierbare plattenförmige Element benachbart ist, erfasst, insbesondere jeweils mittels eines Wärmestromsensors, beispielsweise eines flächig ausgebildeten Wärmestromsensors. Dies ermöglicht eine zuverlässige, genaue und direkte Erfassung des Wärmestroms. Die beidseitige Erfassung des Wärmestroms kann beispielsweise auch zur Kontrolle der Symmetrie der Betriebsweise herangezogen werden.

Gemäß einer Ausgestaltung werden die Wärmestromsensoren jeweils flächig in einem Mittenbereich einer der Hauptoberflächen der platten- oder scheibenartigen Geometrie zwischen dem dieser Hauptoberfläche benachbarten temperierbaren plattenförmigen Element und der Probe angeordnet. Insbesondere kann der Wärmestromsensor jeweils einen großen Mittenbereich der Hauptoberfläche einnehmen. Der vom Wärmestromsensor eingenommene Mittenbereich der Hauptoberfläche kann bei einigen beispielhaften Ausgestaltungen in der Hauptoberfläche betrachtet eine Größe von mindestens einem Drittel oder mindestens der Hälfte der Abmessung der Hauptoberfläche in jeder von zwei, insbesondere orthogonalen, Koordinatenrichtungen aufweisen. Der von dem Wärmestromsensor als Messfläche eingenommene Mittenbereich kann in einigen beispielhaften Ausgestaltungen insbesondere mindestens ein Neuntel oder mindestens ein Sechstel oder mindestens ein Viertel des Flächeninhalts der Hauptoberfläche einnehmen. Auf diese Weise kann erreicht werden, dass ein Einfluss von Randeffekten aufgrund der endlichen Größen der Probe und der temperierbaren plattenförmigen Elemente, die sich kaum zur Gänze vermeiden lassen, vernachlässigbar gering ist oder nicht vorhanden ist. Eine flächige Ausdehnung des Wärmestromsensors in dem genannten großen Mittenbereich ermöglicht es, für eine gute Aussage über das Probematerial einen großen Teil desselben einzubeziehen. Eine weitere Vergrößerung des von dem Wärmestromsensor als aktiver Messfläche eingenommenen Anteils der Hauptoberfläche über die vorgenannten Werte hinaus kann vorteilhaft sein, um eine noch bessere Mittelung über die möglicherweise inhomogene Probe zu erzielen.

In einer Weiterbildung wird/werden eines oder beide der temperierbaren plattenförmigen Elemente vor dem Herbeiführen der Temperaturänderung der Probe auf die Probe zu bewegt, wobei vermittelt über die temperierbaren plattenförmigen Elemente eine vordefinierte Kraft auf die Probe in einer Dickenrichtung der platten- oder scheibenartigen Geometrie aufgebracht oder eine vordefinierte Dicke der Probe in der Dickenrichtung der platten- oder scheibenartigen Geometrie eingestellt wird. Auf diese Weise können definierte Bedingungen, wie beispielsweise eine definierte Dicke der Probe, für die durchzuführende Untersuchung geschaffen, ein zuverlässiger Kontakt mit dem Probematerial für eine definierte Temperierung erreicht und für einen möglichst geringen und gleichmäßigen Wärmeübergangswiderstand an der Kontaktfläche gesorgt werden.

Insbesondere ist in einer Ausgestaltung vorgesehen, dass das Probematerial mit einer Umhüllung versehen wird, die das Probematerial in einem abgeschlossenen Probevolumen einschließt. Alternativ hierzu kann in einer anderen Ausgestaltung insbesondere vorgesehen sein, dass das Probematerial randseitig umlaufend vom einem sich in der Dickenrichtung der Probe im Wesentlichen über die Dicke oder Einfüllhöhe der Probe erstreckenden, flexiblen Dichtelement umgeben wird, welches dafür ausgebildet ist, gemeinsam mit den temperierbaren plattenförmigen Elementen das abgeschlossene Probevolumen einzuschließen, wobei das Dichtelement insbesondere eingerichtet ist, mit den temperierbaren plattenförmigen Elementen jeweils in einen dichtenden Kontakt zu treten. Indem die Umhüllung oder das Dichtelement als eine Art Rand vorgesehen wird, kann ein Abfließen des sich bei einem Aufschmelzen verflüssigenden Probematerials verhindert werden. Vorzugsweise ermöglicht das Dichtelement mittels seiner Flexibilität noch ein Nachfahren durch Bewegen des oder der plattenförmigen Elemente(s), um beim Aufschmelzen einen Kontakt des Probematerials mit den plattenförmigen Elementen aufrecht zu halten.

Gemäß einer Ausgestaltung ist vorgesehen, dass die Temperatur der Probe an einer Oberfläche der Probe oder in einem Inneren der Probe erfasst wird und/oder dass die Temperatur des temperierbaren plattenförmigen Elements jeweils an einer Oberfläche desselben oder in einem Inneren desselben erfasst wird.

Bei einer Weiterbildung wird das Vergleichen der Kurvenbeschaffenheit und der Referenzkurvenbeschaffenheit unter Verwendung korrespondierender, die Kurvenform und die Referenzkurvenform jeweils zumindest abschnittsweise charakterisierender Abszissen- und/oder Ordinatenwerte durchgeführt.

Bei einer weiteren Weiterbildung wird das Vergleichen der Kurvenbeschaffenheit und der Referenzkurvenbeschaffenheit anhand mindestens einer charakteristischen Stufe und/oder mindestens eines charakteristischen Extremums, insbesondere Peaks, jeweils des erhaltenen Verlaufs des Wärmestroms oder der zeitlichen Änderung der Temperatur sowie des Referenzverlaufs durchgeführt, wobei die Stufe oder das Extremum jeweils insbesondere einer Umwandlung innerhalb des Probematerials korrespondiert.

In einer Ausgestaltung wird für mindestens eine Stufe oder mindestens ein Extremum, insbesondere einen Peak, des erhaltenen Verlaufs des Wärmestroms oder der zeitlichen Änderung der Temperatur mindestens ein Abszissenwert, der einem der Stufe oder dem Extremum innerhalb der Kurvenform zugeordneten Anfangspunkt oder Endpunkt oder Mittelpunkt oder Wendepunkt oder Extremalwert korrespondiert, ermittelt und mit mindestens einem korrespondierenden Abszissenwert mindestens eines Referenzverlaufs verglichen.

Gemäß einer weiteren Ausgestaltung ist vorgesehen, dass für mindestens eine Stufe des erhaltenen Verlaufs des Wärmestroms oder der zeitlichen Änderung der Temperatur eine Höhe der Stufe ermittelt und mit einer Höhe mindestens einer Stufe mindestens eines Referenzverlaufs verglichen wird, und/oder dass für mindestens ein Extremum, insbesondere einen Peak, des erhaltenen Verlaufs des Wärmestroms oder der zeitlichen Änderung der Temperatur der zugeordnete Extremalwert des Wärmestroms oder der zeitlichen Änderung der Temperatur ermittelt und mit einem Extremalwert mindestens eines Extremums, insbesondere Peaks, mindestens eines Referenzverlaufs verglichen wird.

Eine Auswertung der Kurvenbeschaffenheit gemäß den vorstehenden Ausführungen und Weiterbildungen, die zur weiteren Verbesserung kombinierbar sind, ermöglicht eine zuverlässige Identifizierung der Komponente(n).

Bei einer Weiterbildung wird mindestens ein charakteristischer Integralwert, insbesondere eine Fläche unter mindestens einem Abschnitt des als Kurve dargestellten, erhaltenen Verlaufs des Wärmestroms oder der zeitlichen Änderung der Temperatur, beispielsweise unter einem Peak, ermittelt und mit einem charakteristischen Integralwert des Referenzverlaufs verglichen. Bei dieser Weiterbildung wird das Identifizieren der mindestens einen Komponente des Probematerials zusätzlich auf dem Ergebnis des Vergleichs der charakteristischen Integralwerte basierend durchgeführt. Dies kann zu einer weiteren Verbesserung der Identifizierung beitragen. Der Integralwert kann einem Enthalpieunterschied, etwa bei einem Phasenübergang in der Probe, entsprechen oder einem Unterschied in der spezifischen Wärmekapazität, etwa bei einem Glasübergang in der Probe, korrespondieren.

In einer weiteren Ausgestaltung wird der erhaltene Verlauf des Wärmestroms oder der zeitlichen Änderung der Temperatur mit dem Referenzverlauf jeweils in deren Gesamtheit direkt verglichen.

Bei einer Weiterbildung umfasst das Vergleichen der Kurvenbeschaffenheit, insbesondere Kurvenform, und der Referenzkurvenbeschaffenheit, insbesondere Referenzkurvenform, den Einsatz eines Mustererkennungsalgorithmus, beispielsweise eines lernfähigen Algorithmus, und/oder eines Verfahrens künstlicher Intelligenz.

Mit einem direkten Vergleich und/oder Einsatz von Musterkennung oder künstlicher Intelligenz kann die Genauigkeit und Zuverlässigkeit der Identifizierung noch weiter vorangetrieben werden.

Gemäß einer Ausgestaltung werden als Referenzverläufe eine Vielzahl jeweils für ein bekanntes Probematerial oder Probematerialgemisch vorab ermittelter Verläufe des Wärmestroms oder der zeitlichen Änderung der Temperatur verwendet.

Gemäß einer Ausgestaltung werden die Referenzverläufe in Form einer Datensammlung bereitgestellt, welche Referenzverläufe einer anwendungsspezifischen Auswahl an Reinstoffen und Gemischen, beispielsweise Kunststoffen und Kunststoffgemischen, beinhaltet.

Indem auf eine Vielzahl von Referenzverläufen, beispielsweise anhand der Datensammlung, die zum Beispiel in Form einer Datenbank bereitgestellt sein kann, zurückgegriffen werden kann, wird eine zuverlässige Aussage über einen weiten Bereich von möglichen Beimischungen oder Verunreinigungen einer Probe möglich. Ein derartiges Verfahren kann sich beispielsweise zur Kontrolle von Recyclingprozessen, bei denen naturgemäß vielfach kaum eine vollständige Gewissheit über die Ausgangsmaterialien zu erlangen ist, als sehr nützlich erweisen.

Die Datensammlung, etwa Datenbank, kann zum Beispiel auf einem geeigneten Speichermedium bereitgestellt oder über ein Netzwerk abrufbar sein.

Bei einer Weiterbildung wird das Probematerial als ein loses Material mit einer Vielzahl einzelner Stücke bereitgestellt, beispielsweise als ein Granulat oder ein Pulver.

Bei einer anderen Weiterbildung wird das Probematerial als ein zusammenhängender Körper bereitgestellt.

Eine Bereitstellung als loses Material oder fester Körper ermöglicht den Einsatz bei unterschiedlich gearteten Proben.

Gemäß einer Ausgestaltung erfolgt die Erfassung des Verlaufs des Wärmestroms oder der Temperatur in Abwesenheit einer gleichzeitigen Referenzmessung, insbesondere an einer geometrisch mit der Probe übereinstimmenden, mit einem vorab bekannten Material gebildeten Referenz oder durch Leermessung als Referenz, innerhalb derselben Untersuchungsanordnung oder mittels einer weiteren identischen Untersuchungsanordnung. Auf diese Weise werden sowohl der apparative Aufwand als auch der Handhabungsaufwand bei der Untersuchung der Probe erheblich verringert, was zu einer Verminderung von Kosten und Zeitaufwand beiträgt. Ein leerer "Zwilling" der Untersuchungsanordnung als Referenz beispielsweise wird nicht benötigt. Eine einfache, zielgerichtete Untersuchung des Probematerials unter Vermeidung von eventuellen Fehlerquellen wird somit möglich.

In einer Ausgestaltung weist die untersuchte Probe eine Masse von mehr als etwa 10 Gramm, beispielsweise zwischen etwa 10 Gramm und etwa 10 Kilogramm, bevorzugt zwischen etwa 10 Gramm und etwa 1 Kilogramm, weiter bevorzugt zwischen etwa 10 Gramm und etwa 200 Gramm, beispielsweise zwischen etwa 10 Gramm und etwa 100 Gramm, auf. Derartige Proben machen es möglich, ein zuverlässiges und repräsentatives Bild über eine größere Materialcharge, wie sie im industriellen Maßstab, beispielsweise in der Recyclingbranche, vorkommt, zu gewinnen.

Gemäß einer Weiterbildung ist vorgesehen, dass das Probematerial ein Gemisch ist und mittels des Verfahrens ein Material oder mehrere Materialien, das/die in der Probe enthalten ist/sind, identifiziert wird/werden.

In einer vorteilhaften Ausgestaltung wird das Verfahren zur Reinheitsbestimmung eines Kunststoffrecyclats verwendet, insbesondere zur Identifizierung eventueller Beimischungen oder Verunreinigungen und/oder zur Bestimmung eines Ausmaßes dieser.

Insbesondere kann das Verfahren in einer Prozesskette beim Kunststoff-Recycling verwendet werden. Somit kann in zeitsparender und einfacher Weise eine Kontrolle des Recyclingvorgangs in verschiedenen Stufen desselben, sowie eine Qualitätskontrolle des erhaltenen Endprodukts, etwa eines Recyclingkunststoffgranulats, ermöglicht werden.

In einer beispielhaften Weiterbildung wird als das Probematerial der Probe ein compoundiertes Stoffgemisch, insbesondere ein compoundierter Kunststoff, bereitgestellt. Die Erfindung ermöglicht es vorteilhaft, auch einen derart bereits aufbereiteten Kunststoff hinsichtlich seiner Komponenten zu untersuchen.

### KURZBESCHREIBUNG DER ZEICHNUNGEN

Die Erfindung wird ausführlicher mit Verweis auf beispielhafte Ausführungsformen beschrieben, die in den beiliegenden Zeichnungen dargestellt sind.

Die beiliegenden Zeichnungen sind enthalten, um ein weiteres Verständnis dieser Erfindung zu ermöglichen und sind in diese Beschreibung einbezogen und stellen einen Teil davon dar. Die Zeichnungen illustrieren die Ausführungsformen dieser Erfindung und dienen gemeinsam mit der Beschreibung der Erklärung der Grundsätze der Erfindung. Andere Ausführungsformen dieser Erfindung und viele der vorgesehenen Vorteile dieser Erfindung sind leicht zu verstehen, wenn sie durch Verweis auf die folgende ausführliche Beschreibung besser verständlich werden. Die Elemente der Zeichnungen sind nicht notwendigerweise im gleichen Maßstab zueinander gezeichnet. Gleiche Bezugszeichen bezeichnen entsprechend ähnliche Teile.
- Fig. 1: zeigt eine schematische Querschnittsansicht einer Anordnung, die zur Durchführung eines Verfahrens gemäß Ausführungsbeispielen der Erfindung verwendet wird;
- Fig. 2: zeigt perspektivisch und schematisch eine platten- oder scheibenartige Geometrie des zur Durchführung des Verfahrens gemäß den Ausführungsbeispielen verwendeten Probematerials oder eines Raumgebiets, in dem das Probematerial zur Durchführung des Verfahrens gemäß den Ausführungsbeispielen angeordnet wird;
- Fig. 3: zeigt ein beispielhaftes Messergebnis, das mittels der in Fig. 1 schematisch dargestellten Anordnung erhalten und gemäß einem Ausführungsbeispiel zum Identifizieren mindestens einer Komponente eines Probematerials verwendet wird, beispielhaft gemessen für eine Probe eines Polylactids (PLA);
- Fig. 4: zeigt ein beispielhaftes Messergebnis für ein Polylactid (PLA), das mittels eines konventionellen Verfahrens der dynamischen Differenzkalorimetrie (DSC) erhalten wurde, wobei zwei Aufheizungen beispielhaft gezeigt sind;
- Fig. 5: zeigt ein beispielhaftes Messergebnis, das mittels der in Fig. 1 schematisch dargestellten Anordnung erhalten und gemäß einem weiteren Ausführungsbeispiel zum Identifizieren mindestens einer Komponente eines Probematerials verwendet wird, beispielhaft gemessen für eine Probe eines Paraffins;
- Fig. 6: zeigt ein beispielhaftes Messergebnis für ein Paraffin, das mittels eines konventionellen Verfahrens der dynamischen Differenzkalorimetrie (DSC) erhalten wurde;
- Fig. 7: zeigt ein schematisches Ablaufdiagramm eines Verfahrens gemäß Ausführungsbeispielen der Erfindung;
- Fig. 8: illustriert schematisch eine beispielhafte Umhüllung für das Probematerial zur Durchführung von Verfahren gemäß Ausführungsbeispielen der Erfindung; und
- Fig. 9: illustriert schematisch ein randseitiges Dichtelement zum Einschließen des Probematerials, zur Durchführung von Verfahren gemäß weiteren Ausführungsbeispielen.

In den Figuren bezeichnen gleiche Bezugszeichen gleiche oder funktional ähnliche Bauteile, wenn nicht anders angegeben. Alle Richtungsbezeichnungen, wie "oben", "unten", "links", "rechts", "über", "unter", "horizontal", "vertikal", "hinten", "vorne" und ähnliche Begriffe werden nur zur Erklärungszwecken verwendet und sollen die Ausführungsformen nicht auf die spezifischen Anordnungen beschränken, die in den Zeichnungen dargestellt sind.

### AUSFÜHRLICHE BESCHREIBUNG DER ERFINDUNG

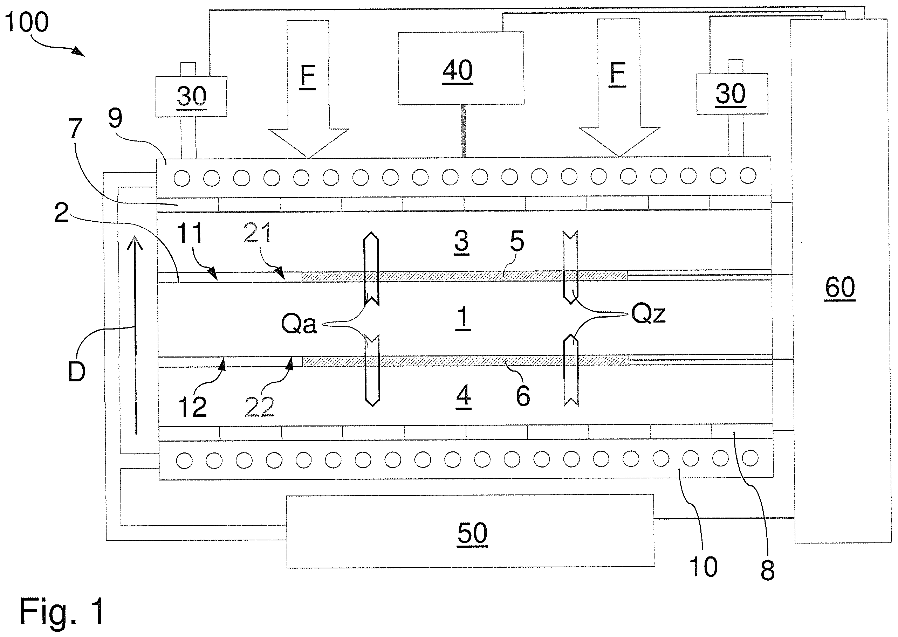

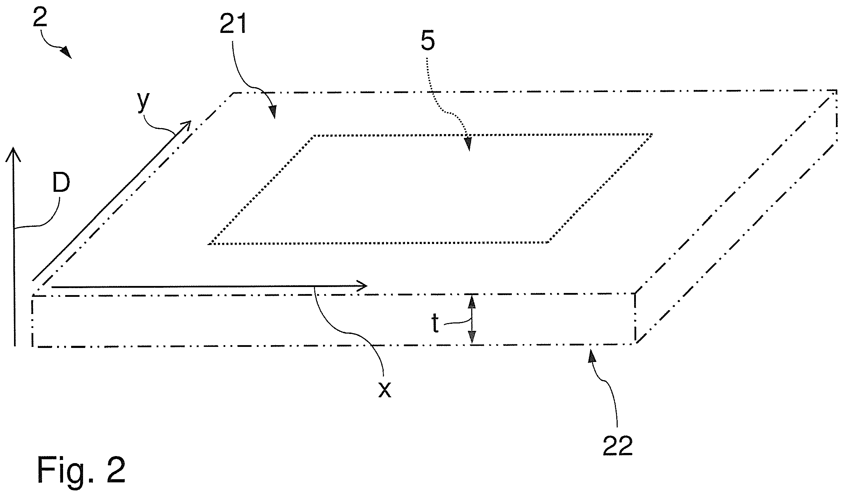

Fig. 1 zeigt eine Anordnung 100, die zur Durchführung von Verfahren gemäß nachfolgend erläuterten Ausführungsbeispielen der Erfindung verwendet werden kann. Die Anordnung 100 dient der Untersuchung einer Probe 1. Zur Bereitstellung der Probe 1 wird ein Probematerial, das beispielsweise einer Charge eines aus einem Recyclingprozess erhaltenen, losen Kunststoffgranulats entnommen ist, in einem Raumgebiet mit einer platten- oder scheibenartigen Geometrie 2 angeordnet. Hierzu kann das Probematerial in einen nicht näher dargestellten Proberahmen gefüllt werden, der die Geometrie 2 definiert. Alternativ kann es sich bei dem Probematerial der Probe 1 um einen zusammenhängenden festen Körper oder alternativ um einen kompressiblen Körper, der jeweils die platten- oder scheibenartigen Geometrie 2 aufweist, beispielsweise entsprechend zugeschnitten ist, handeln. Fig. 2 zeigt beispielhaft eine derartige platten- oder scheibenförmige Geometrie 2 in schematischer Weise. Die Geometrie 2 ist in Fig. 2 flach und quaderartig ausgebildet, wobei Erstreckungen der Geometrie 2 in den beiden orthogonal zueinander stehenden Koordinatenrichtungen x und y wesentlich größer sind als eine Erstreckung oder Dicke t der Geometrie 2 in der zu x und y senkrechten Dickenrichtung D. Hauptoberflächen 21 und 22 der Geometrie 2 sind von im Wesentlichen gleicher Form und Größe sowie parallel ausgebildet, und bei dem gezeigten Ausführungsbeispiel in der Fig. 2 beispielhaft rechteckig.

Das Probematerial der Probe 1, unabhängig von dessen physischer Form etwa als Granulat oder zusammenhängender Körper oder anders, ist insbesondere ein Gemisch, mit mindestens einem Hauptmaterial, dem aufgrund des Recyclingsprozesses ein oder mehr andere Materialien beigemischt sein können, insbesondere als Verunreinigung des möglichst rein gewünschten Hauptmaterials. Das Probematerial umfasst somit Komponenten, wobei sich dieser Begriff sowohl auf den/die Hauptbestandteil(e) als auch auf eventuelle unerwünschte Beimengungen bezieht.

Bei der Anordnung 100 der Fig. 1 ist die Probe 1 sandwichartig zwischen zwei temperierbaren plattenförmigen Elementen 3 und 4 angeordnet. Ferner weist die Anordnung 100 einen zwischen der Hauptoberfläche 21 der Geometrie 2 und dem plattenförmigen Element 3 angeordneten Wärmestromsensor 5 und einen zwischen der Hauptoberfläche 22 der Geometrie 2 und dem plattenförmigen Element 4 angeordneten Wärmestromsensor 6 auf.

Ein Wärmestrom, der durch die Hauptoberflächen 21, 22 von dem temperierbaren Element 3 bzw. 4 in das Probematerial hinein bzw. aus diesem heraus in das Element 3 bzw. 4 fließt, kann bei der Anordnung 100 der Fig. 1 auf beiden temperierten Seiten 11, 12 der Probe 1 mittels der Wärmestromsensoren 5 und 6 (im Englischen als heat flux transducer bezeichnet) gemessen werden. Die Seite 11 der Probe 1 ist hierbei dem temperierbaren Element 3, die Seite 12 dem temperierbaren Element 4 zugewandt. Der Wärmestromsensor 5, 6 ist dafür ausgebildet, einen durch ihn fließenden Wärmestrom zu detektieren. Zum Beispiel kann der Wärmestromsensor 5, 6 ein dem hindurchfließenden Wärmestrom proportionales Ausgangssignal liefern, aus dem nach Kalibrierung ein zahlenmäßiger Absolutwert für den Wärmestrom erhalten werden kann.

Die Wärmestromsensoren 5 und 6 sind jeweils flächig ausgebildet und jeweils in einem Mittenbereich der zugeordneten Hauptoberfläche 21 oder 22 angeordnet. Beispielhaft ist ein Umriss des Wärmestromsensors 5 schematisch in Fig. 2 in punktierter Linie angedeutet, wobei der Wärmestromsensor 6 im Bereich der entgegengesetzten Hauptoberfläche 22 analog und symmetrisch zum Sensor 5 angeordnet ist.

Jeder der Wärmestromsensoren 5, 6 nimmt einen vergleichsweisen großen Mittenbereich der Hauptoberfläche 21 bzw. 22 ein, der beispielsweise in der Hauptoberfläche 21 oder 22 betrachtet eine Größe von mindestens einem Drittel der Abmessung der Hauptoberfläche 21 oder 22 in jeder der Koordinatenrichtungen x und y aufweist. Dies hilft, den Einfluss von Randeffekten zu minimieren und zugleich den Wärmestrom durch einen großen Bereich des Probematerials zu erfassen. Beispielhaft nimmt in Fig. 2 jeder Wärmestromsensor 5, 6 einen Mittenbereich als aktive Messfläche ein, der in etwa einem Viertel des Flächeninhalts der Hauptoberfläche 21 oder 22 entspricht. Andere, insbesondere größere Flächenanteile sind jedoch zur weiteren Verbesserung der Mittelung über die Probe 1 vorstellbar.

Die Anordnung 100 umfasst ferner einen dem temperierbaren plattenförmigen Element 3 zugeordneten, sich parallel zu dem Element 3 erstreckenden Kühlkörper 9 sowie einen dem temperierbaren plattenförmigen Element 4 zugeordneten, sich parallel zu dem Element 4 erstreckenden Kühlkörper 10. Die Kühlung der Kühlkörper 9, 10 selbst erfolgt mittels eines Kühlsystems 50.

Weiterhin ist jeweils zwischen dem Kühlkörper 9 und dem Element 3 sowie dem Kühlkörper 10 und dem Element 4 ein Temperiersystem 7 bzw. 8 zur Temperierung angeordnet. Beispielsweise kann das Temperiersystem 7, 8 jeweils als eine oder mit einer Heizeinrichtung, wie etwa eine(r) Widerstands-Heizeinrichtung, ausgebildet sein. Mit einer derartigen Heizeinrichtung kann beispielsweise ein Temperaturbereich, der bei einer Anwendung der Erfindung auf das Gebiet des Kunststoffrecyclings vorteilhaft ist und zum Beispiel Temperaturen von 200 Grad Celsius oder mehr einschließen kann, gut bestrichen werden. Bei anderen Anwendungen der Erfindung, in denen niedrigere Temperaturen zum Einsatz kommen, kann das Temperiersystem 7, 8 jeweils alternativ als ein Peltiersystem ausgebildet sein. Ein Peltiersystem kann den Vorteil bieten, dass es sowohl ein Heizen als auch ein Kühlen ermöglicht.

Eine mit dem Kühlkörper 9, dem Temperiersystem 7 und dem plattenförmigen Element 3 gebildete Baugruppe ist zudem mittels Verfahreinrichtungen 30 im Wesentlichen senkrecht zu einer Haupterstreckungsebene der Probe 1 und somit senkrecht zu den Hauptoberflächen 21, 22 verfahrbar. Mittels der Einrichtungen 30 kann eine Kraft F in Dickenrichtung D auf die Probe 1 aufgebracht werden. Ein Verfahrweg oder eine Dicke t der Probe 1 können mittels einer Dickenmesseinrichtung 40 in Dickenrichtung D der Probe 1 erfasst werden. In einer Variante können beide Elemente 3 und 4 nebst jeweils zugeordnetem Kühlkörper 9, 10 und Temperiersystem 7, 8 aufeinander und auf die Probe 1 zu verfahrbar sein.

Die Temperiersysteme 7 und 8, das Kühlsystem 50, die Verfahreinrichtungen 30, die Dickenmesseinrichtung 40 und die Wärmestromsensoren 5, 6, sowie ggf. zusätzliche Temperatursensoren, etwa Thermoelemente, die an den unten noch näher beschriebenen Stellen in unterschiedlicher Kombination vorgesehen sein können, sind mit einem Datenverarbeitungs- und Steuerungssystem 60 gekoppelt, das es ermöglicht, insbesondere die Einrichtungen und Systeme 30, 50, 7, 8 anzusteuern und mittels der Einrichtung 40 und der Wärmestromsensoren 5, 6 sowie der genannten Temperatursensoren erfasste Messwerte zu verarbeiten. Das System 60 kann zudem mit nicht dargestellten Eingabe- und Ausgabeeinrichtungen, Speichern sowie weiteren Einrichtungen gekoppelt sein. Auch ist es denkbar, das System 60 mit einer Netzwerkschnittstelle zu koppeln.

Wenngleich bei dem Ausführungsbeispiel der Fig. 1 die Hauptoberflächen 21, 22 und somit die Haupterstreckungsebenen der Probe 1 sowie der Elemente 3, 4 beispielhaft horizontal ausgerichtet sind, ist eine vertikale Ausrichtung oder eine sonstige Ausrichtung im Raum ebenfalls vorstellbar.

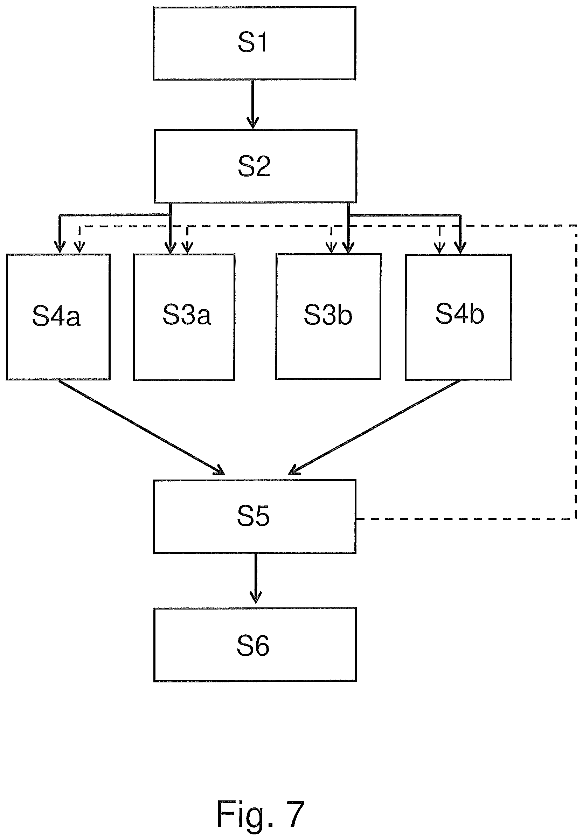

Nachfolgend werden Verfahren gemäß Ausführungsbeispielen der Erfindung, ausgehend von einer in Fig. 7 gegebenen schematischen Darstellung des Verfahrensablaufs, erläutert und ferner beispielhafte, mit derartigen Verfahren erhaltene Ergebnisse, sowie zum Vergleich Ergebnisse herkömmlicher DSC-Messungen, besprochen.

Fig. 7 zeigt ein Verfahren zum Identifizieren mindestens einer Komponente des Probematerials, mit dem die Probe 1 gebildet ist.

In einem ersten Schritt S1 des Verfahrens wird die Probe 1, wie oben beschrieben, bereitgestellt und hierbei das Probematerial in einem Raumgebiet mit der platten- oder scheibenartigen Geometrie 2, siehe Fig. 2, angeordnet, beispielsweise als ein loses Material, etwa das oben genannte Granulat. Alternativ hierzu wird im Schritt S1 das Probematerial der Probe 1 mit der platten- oder scheibenartigen Geometrie 2 ausgebildet, beispielsweise als kompakter fester Körper oder als ein kompressibler Körper. Eine Masse der Probe 1 ist größer als etwa 10 Gramm und liegt beispielsweise in einem Bereich zwischen etwa 10 Gramm und etwa 10 Kilogramm, bevorzugt zwischen etwa 10 Gramm und etwa 1 Kilogramm, weiter bevorzugt zwischen etwa 10 Gramm und etwa 200 Gramm. Eine weitere bevorzugte Masse der Probe 1 liegt beispielsweise zwischen etwa 10 Gramm und etwa 100 Gramm. Je nach Masse der Probe 1 kann die Dimensionierung der Anordnung 100 entsprechend, etwa hinsichtlich der Abmessungen, der mechanischen Stabilität und der Heiz- und Kühlleistungen, angepasst sein.

In einem zweiten Schritt S2 wird die Probe 1 zwischen den beiden temperierbaren plattenförmigen Elementen 3 und 4 angeordnet. Dies wird derart durchgeführt, dass sich die temperierbaren plattenförmigen Elemente 3 und 4 entlang jeweils einer der beiden entgegengesetzten, im Wesentlichen ebenen Hauptoberflächen 21 und 22 der Geometrie 2 erstrecken. Die Probe 1 und die plattenförmigen Elemente 3 und 4 werden somit im Wesentlichen sandwichartig parallel zueinander angeordnet. Hierbei werden zwischen der Hauptoberfläche 21 und dem Element 3 der flächige Wärmestromsensor 5 und zwischen der Hauptoberfläche 22 und dem Element 4 der flächige Wärmestromsensor 6 angeordnet. Die Wärmestromsensoren 5 und 6 sind vorzugsweise jeweils fest in das plattenförmige Element 3 oder 4 integriert und werden mit den Hauptoberflächen 21, 22 in Kontakt gebracht, nachdem die Probe 1 zwischen die Elemente 3, 4 verbracht wurde.

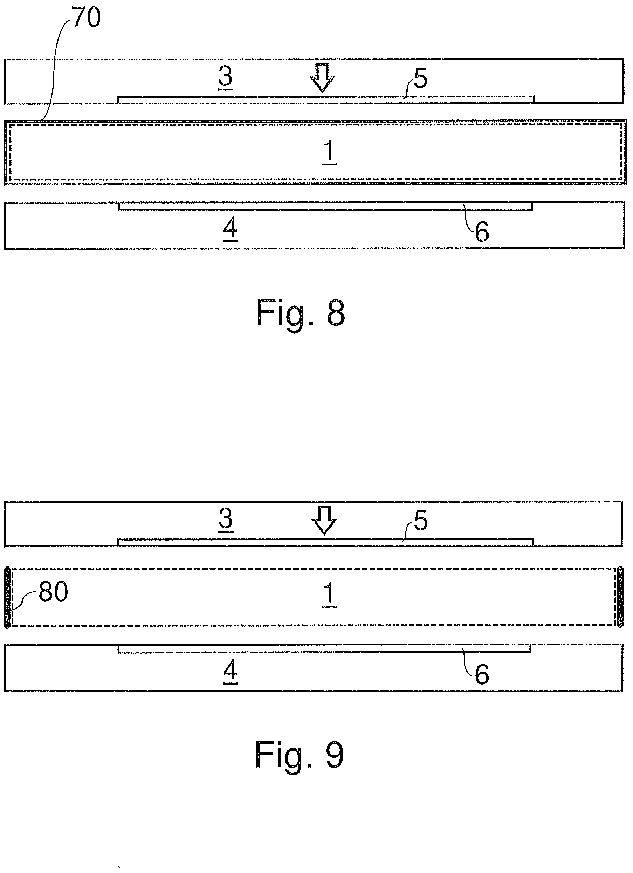

Damit das Probematerial bei einem nachfolgenden Erwärmen und Aufschmelzen oder Verflüssigen, bei einem Phasen- oder Glasübergang, nicht ausläuft, wird das Probematerial der Probe 1 in Ausführungsbeispielen mit einer Umhüllung 70 oder einem flexiblen Dichtelement 80 umgeben, siehe Fig. 8 und 9, in denen die Umhüllung 70 bzw. das Dichtelement 80 zusammen mit den von der Probe 1 noch beabstandeten Elementen 3, 4 zur schematischen Illustration gezeigt sind. Die Umhüllung 70 oder das Dichtelement 80 können alternativ in der Anordnung 100 der Fig. 1 verwendet werden, um die Verfahren der hier beschriebenen Ausführungsbeispiele durchzuführen, sind in Fig. 1 jedoch der besseren Übersicht halber nicht gezeigt.

Die Umhüllung 70 in Fig. 8 umgibt das Probematerial der Probe 1 vollständig und kapselt dieses in ein abgeschlossenes Volumen ein. Die Umhüllung 70 kann mit einem flexiblen Material gebildet sein. Hingegen umgibt das Dichtelement 80 der Fig. 9 das Probematerial der Probe 1 randseitig und rahmenartig entlang des Umfangs der Probe 1, wobei das Dichtelement 80 mit jedem der temperierbaren plattenförmigen Elemente 3, 4 dichtend in Kontakt treten kann.

In dem Schritt S2 wird, nach Anordnen der Probe 1 zwischen den Elementen 3, 4 in der soeben beschriebenen Weise, die in Fig. 1 obere Konfiguration mit dem plattenförmigen Element 3 sowie dem diesem zugeordneten Temperiersystem 7 und dem Kühlkörper 9, mittels der Verfahreinrichtungen 30 auf die Probe 1 zu bewegt. Hierdurch kann ein definierter Kontakt mit geringem, gleichmäßigem Wärmeübergangswiderstand an den Hauptoberflächen 21, 22 und, bei kompressiblen und/oder losen Materialien, eine definierte Dicke t des Probematerials erzielt werden. Eine definierte Einstellung und Kontrolle der Dicke t wird mittels der Dickenmesseinrichtung 40 ermöglicht. Vorzugsweise werden hierbei die Verfahreinrichtungen 30 durch das Datenverarbeitungs- und Steuerungssystem 60 angesteuert, das zudem mit der Messeinrichtung 40 verbunden ist und deren erfasste Wegstrecken- oder Dickenwerte aufnimmt und bei der Steuerung der Verfahreinrichtungen 30 berücksichtigt sowie diese Werte anzeigen und/oder zur Dokumentation speichern kann. Mittels der Verfahreinrichtungen 30 kann hierbei eine vordefinierte Kraft oder Last F auf die Probe 1 aufgebracht werden. Die Last F kann beispielsweise mittels einer in Fig. 1 nicht dargestellten Kraftmesseinrichtung erfasst und mit einem Sollwert abgeglichen werden. Wird das Dichtelement 80 verwendet, so erfolgt das soeben beschriebene Verfahren mittels der Verfahreinrichtungen 30 derart, dass das Dichtelement 80 an beiden Elementen 3 und 4 dichtend zur Anlage gelangt.

In einem dritten Schritt S3a oder S3b wird eine Temperaturänderung der Probe 1 herbeigeführt. Hierzu wird in einer symmetrischen Weise ein Wärmezufluss Qz in die Probe 1 hinein oder ein Wärmeabfluss Qa aus der Probe 1 heraus durch die beiden Hauptoberflächen 21 und 22 der platten- oder scheibenartigen Geometrie 2 herbeigeführt. Durch die symmetrische Betriebsweise der Anordnung 100 kann somit, im Rahmen der mit den Komponenten der Anordnung 100 erreichbaren Genauigkeit und bei hinreichender Homogenität der Probe 1, jeweils im Wesentlichen die Hälfte des Gesamtwärmestroms über eines der temperierbaren, plattenförmigen Elemente 3 und 4 zu- oder abfließen.

Die Temperaturänderung der Probe 1 wird mittels Temperierens der plattenförmigen Elemente 3, 4 mit Hilfe des jeweils vorgesehenen Temperiersystems 7, 8 und des jeweils vorgesehenen Kühlkörpers 9, 10 bewirkt. Hierzu werden das Temperiersystem 7, 8 und das mit den Kühlkörpern 9, 10 verbundene Kühlsystem 50 mittels des Datenverarbeitungs- und Steuerungssystems 60 angesteuert.

Die im dritten Schritt S3a oder S3b bewirkte Temperaturänderung bestreicht einen Temperaturbereich, innerhalb dessen mindestens eine endotherme oder exotherme Veränderung der mindestens einen Komponente des Probematerials auftritt. Eine derartige Veränderung ist vorzugsweise ein Phasenübergang, beispielsweise ein Schmelzen oder Gefrieren mindestens einer Komponente oder mehrerer Komponenten des Probematerials, oder ein Glasübergang einer oder mehrerer solcher Komponente(n), oder sowohl ein Phasen- als auch ein Glasübergang, sofern eine Komponente beispielsweise kristalline und amorphe Anteile aufweist.

Um die Temperaturänderung herbeizuführen, sind gemäß alternativen Ausführungsbeispielen der Erfindung zwei verschiedene Wege vorgesehen. Dies ist in Fig. 7 mittels zweier alternativer Schritte S3a oder S3b verdeutlicht.

Gemäß Schritt S3a wird die Temperaturänderung in der Weise durchgeführt, dass das Probematerial einem vordefinierten, beispielsweise in einer nicht dargestellten Speichereinrichtung, auf die das Datenverarbeitungs- und Steuerungssystem 60 zugreifen kann, hinterlegten Temperaturverlauf unterzogen wird. Dieser Temperaturverlauf ist kontinuierlich und insbesondere als eine vordefinierte, linear mit der Zeit ansteigende oder abfallende Temperaturrampe gewählt, die den oben genannten Temperaturbereich bestreicht. Die Temperatur wird hierbei beispielsweise mittels eines Temperatursensors bzw. Temperatursensoren an einer Hauptoberfläche 21, 22 der Probe 1 oder beiden Hauptoberflächen 21, 22 erfasst oder die Temperatur wird im Inneren der Probe 1 erfasst. Alternativ oder zusätzlich ist eine Erfassung der Temperatur mittels Temperatursensor(en) an einer der Hauptoberfläche 21 bzw. 22 zugewandten Oberfläche des plattenförmigen Elements 3 oder 4 oder beider oder im Inneren eines oder beider plattenförmiger Elemente 3, 4 möglich. Der vorzuschreibende Temperaturverlauf kann der Temperatur an einer dieser Stellen korrespondieren oder einer Temperatur korrespondieren, die zum Beispiel basierend auf der/den Temperatur(en) an einer oder mehrerer der genannten Temperaturmessstellen modellierbar ist. Im Schritt S3a wird die Temperaturänderung symmetrisch von beiden Seiten 11, 12 der Probe 1 eingeprägt. Bei derartiger symmetrischer Temperierung der Elemente 3, 4 und somit symmetrischer Betriebsweise und symmetrischer Herbeiführung einer Temperaturänderung der Probe 1 kann sich, bei ausgeprägter Inhomogenität der Probe 1, aufgrund unterschiedlicher Wärmekapazitäten oder Wärmeleitfähigkeiten von Komponenten des Probematerials ein Unterschied im Wärmestrom, der über das Element 3 und das Element 4 jeweils zu- oder abgeführt wird, ergeben.

Gemäß dem alternativen Schritt S3b wird die Temperaturänderung der Probe in der Weise herbeigeführt, dass ein über der Zeit konstanter Wärmestrom der Probe 1 zugeführt oder aus der Probe 1 abgeführt wird. Dies wird symmetrisch durch Temperieren der temperierbaren, plattenförmigen Elemente 3 und 4 erreicht, wobei der eingetragene oder abgeführte Wärmestrom mittels des Datenverarbeitungs- und Steuerungssystems 60 auf einen vordefinierten konstanten Wert gesteuert oder bevorzugt geregelt wird. Insbesondere wird für einen symmetrischen Betrieb der durch die beiden Elemente 3 und 4 jeweils zu- oder abgeführte Wärmestrom im Schritt S3b auf den gleichen Wert geregelt. Hierzu wird der tatsächlich vorliegende Wärmestrom mit Hilfe der Wärmestromsensoren 5, 6 gemessen. Eine zusätzliche Messung der Temperatur der Probe 1 an den Hauptoberflächen 21, 22 und/oder in dem Inneren der Probe 1, sowie alternativ oder zusätzlich an Oberflächen der plattenförmigen Elemente 3, 4 und/oder in jeweils deren Innerem, wie vorstehend für Schritt S3a beschrieben, ist zur Verbesserung der Regelung des Wärmestroms denkbar.

Während der Temperaturänderungen der Probe 1 gemäß Schritt S3a oder S3b kann ein Nachstellen mittels der Verfahreinrichtungen 30 erfolgen, damit beispielsweise bei einem Phasen- oder Glasübergang mit Verflüssigung ein guter Kontakt zur Probe 1 und ein guter Wärmeübergang zu/von den plattenförmigen Elementen 3 und 4 aufrecht erhalten bleibt.

Während die Temperaturänderung der Probe 1 gemäß Schritt S3a oder S3b vorgenommen wird, wird gleichzeitig in einem vierten Schritt S4a oder alternativ S4b ein Wärmestrom oder eine Temperatur erfasst.

Im Einzelnen wird im Schritt S4a, während die Probe 1 dem definierten, eingeprägten Temperaturverlauf ausgesetzt wird, ein in die Probe 1 hinein oder aus der Probe 1 heraus fließender Wärmestrom mittels der Wärmestromsensoren 5, 6 erfasst. Aus den erfassten Wärmestromwerten wird ein als Kurve darstellbarer Verlauf des Wärmestroms *Q̇*(*T*) oder *Q̇*(*t*) in Abhängigkeit von einer Temperatur T der Probe 1 oder der temperierbaren plattenförmigen Elemente 3, 4, oder stattdessen in Abhängigkeit von der Zeit *t,* erhalten.

Alternativ hierzu wird im Schritt S4b, während der Probe 1 der definierte, konstante Wärmestrom eingeprägt und somit in die Probe 1 eingebracht oder aus dieser abgeführt wird, eine Temperatur der Probe 1 oder der temperierbaren, plattenförmigen Elemente 3, 4 erfasst. Aus den erfassten Temperaturwerten wird ein als Kurve darstellbarer Verlauf einer zeitlichen Änderung der Temperatur *dT*/*dt (t)* oder *dT*/*dt (T)* in Abhängigkeit von der Temperatur oder in Abhängigkeit von der Zeit erhalten.

Die Temperatur der Probe 1 oder der plattenförmigen Elemente 3, 4 kann für Schritt S4a, S4b an den oben zu Schritt S3a und S3b beschriebenen Stellen erfasst werden.

In einem fünften Schritt S5 wird die Kurvenbeschaffenheit des als Kurve darstellbaren, erhaltenen Verlaufs entweder des Wärmestroms *Q̇*(*T*) oder *Q̇*(*t*) oder der zeitlichen Temperaturänderung *dT*/*dt (t)* oder *dT*/*dt (T)* mit einer Referenzkurvenbeschaffenheit einer Referenzkurve, vorzugsweise mehrerer oder weiter bevorzugt einer Vielzahl von Referenzkurven, verglichen.

Dies wird im Folgenden näher beschrieben. Die Referenzkurven werden für die verschiedensten bekannten Probematerialien mittels der gleichen Anordnung 100 und insbesondere unter gleichen Bedingungen und mittels des gleichen Temperatur- oder Wärmestromprogramms sowie gleichen Messstellen wie bei der Untersuchung der eigentlichen Probe 1 jeweils vorab untersucht und die Ergebnisse als Referenzkurven für *Q̇*(*T*) oder *Q̇*(*t*) oder *dT*/*dt (t)* oder *dT*/*dt (T)* vorab in einer Datenbank, auf die das Datenverarbeitungs- und Steuerungssystem 60 zugreifen kann, abgelegt.

Die Referenzkurven können hierbei jeweils für ein bekanntes, einheitliches Probematerial oder ein bekanntes Probematerialgemisch mit zwei oder mehr definierten, bekannten Komponenten oder für eine erwartete, unerwünschte Komponente, etwa eine mögliche Verunreinigung, vorab gemessen und in der Datenbank hinterlegt werden.

Die in der Datenbank verfügbaren Referenzverläufe umfassen vorzugsweise mindestens eine an die jeweilige Anwendung angepasste Auswahl an Reinstoffen und Gemischen, beispielsweise Kunststoffen und Kunststoffgemischen. Die Datenbank kann auf einem geeigneten Speichermedium, in den Figuren nicht dargestellt, bereitgestellt oder über ein Netzwerk von einem entfernten Speichermedium abrufbar sein.

Basierend auf dem Ergebnis des Vergleichs wird die mindestens eine Komponente des Probematerials, oder werden mehrere Komponenten des Probematerials, im sechsten Schritt S6 identifiziert.

In Fig. 7 ist ferner in gestrichelter Linie dargestellt, dass die Schritte S3a, S4a bzw. S3b, S4b wiederholt ausgeführt werden können. Somit kann beispielsweise vorgesehen sein, mittels der temperierbaren plattenförmigen Elemente 3 und 4 mehrere Heiz- und/oder Kühlvorgänge durchzuführen und das Probematerial bzw. die mindestens eine Komponente wiederholt zu erwärmen und abzukühlen, um das Probematerial bzw. die Komponente(n) wiederholt - im Falle eines Phasenübergangs - zu schmelzen oder einzufrieren oder - im Falle eines Glasübergangs - erweichen oder erstarren zu lassen. Auf diese Weise können bei Umwandlungen im Material, die durch die Temperaturänderung über der Zeit erfolgen, je nach Probematerial oder Komponente(n) desselben zusätzliche endotherme oder exotherme Veränderungen, Glasübergänge oder Phasenübergänge erfasst und für die Identifizierung genutzt werden. Hierbei kann beispielsweise unmittelbar nach Erfassen des als Kurve darstellbaren Verlaufs der Schritt S5 des Vergleichs ausgeführt werden, wie beispielhaft in Fig. 7 skizziert, oder alternativ können zunächst alle vorgesehenen Erwärmungs-/Abkühlungsvorgänge ausgeführt, die erhaltenen Kurvenverläufe gespeichert und dann gesammelt der Vergleich im Schritt S5 vorgenommen werden. Hierbei kann zudem vorgesehen sein, dass nicht zwingend die erhaltenen Verläufe für alle Erwärmungs-/Abkühlungsvorgänge für den Vergleich berücksichtigt werden, sondern eine definierte Auswahl vorgesehen ist. Insbesondere kann es vorteilhaft sein, für den Vergleich die zweite Aufheizung heranzuziehen, da diese es möglich macht, von einem einheitlicheren Ausgangszustand auszugehen.

Die Auswertung im Schritt S5 erfolgt durch Auftragung des Wärmestroms *Q̇*(*T*) über der Temperatur oder *Q̇*(*t*) über der Zeit, oder durch Auftragung der Temperaturänderung *dT*/*dt (t)* über der Zeit oder *dT*/*dt (T)* über der Temperatur.

Im Schritt S5 umfasst das Vergleichen der Kurvenbeschaffenheit der für die Probe 1 erfassten und in der vorstehend genannten Weise aufgetragenen Kurve mit der Referenzkurvenbeschaffenheit insbesondere auch einen Vergleich der Kurvenformen der für die Probe 1 aufgetragenen Kurve und der Referenzkurve(n).

Umwandlungen innerhalb des Probematerials äußern sich durch Stufen oder Extrema in diesen Kurvenverläufen. Der Vergleich von Kurvenbeschaffenheit und Referenzkurvenbeschaffenheit kann unter Verwendung korrespondierender, die Kurvenform und die Referenzkurvenform jeweils zumindest abschnittsweise charakterisierender Abszissen- und/oder Ordinatenwerte durchgeführt werden. Durch Ermittlung beispielsweise von Onset-, Endset- und/oder Midpoint-Temperaturen an der für die Probe 1 gemessenen und aufgetragenen Kurve für den Wärmestrom *Q̇*(*t*)*,Q̇*(*T*) oder die Temperaturänderung *dT*/*dt* (*t*), *dT*/*dt* (*T*) bzw. von Abzissenwerten, etwa Temperaturen T, bei denen Extrema auftreten, sowie zusätzlich beispielsweise anhand der integralen Flächen unter Peaks kann auf die Probenzusammensetzung geschlossen werden. Hierzu werden insbesondere die charakteristischen Temperaturen als charakteristische Abszissenwerte, insbesondere die genannten Onset-, Endset-, Midpoint- und Extremaltemperaturen, ggf. die Integralwerte und/oder die Kurvenverläufe in ihrer Gesamtheit, mit den Referenzkurven in der Datenbank sowie für diese Referenzkurven vorab ermittelten korrespondierenden charakteristischen Temperaturen verglichen. Auch die Ordinatenwerte, die den vorgenannten charakteristischen Abszissenwerten zugeordnet sind, können verglichen werden. Die Datenbank beinhaltet Referenzkurven an für die Applikation relevanten Reinstoffen und Gemischen.

Im Zuge der Auswertung im Schritt S5 kann bei einigen Ausführungsbeispielen des Verfahrens beispielsweise ferner vorgesehen sein, dass für mindestens eine Stufe des erhaltenen Verlaufs des Wärmestroms oder der zeitlichen Änderung der Temperatur eine Höhe der Stufe ermittelt und mit einer Höhe mindestens einer Stufe des/der Referenzkurve(n) verglichen wird. Analog kann beispielsweise ein Extremalwert der Kurven von Wärmestrom oder zeitlicher Temperaturänderung selbst als Ordinatenwert mit einem Ordinatenwert eines Extremums der Referenzkurve(n) direkt verglichen werden.

Zudem kann bei der Auswertung im Schritt S5 ein charakteristischer Integralwert im Sinne einer Fläche unter mindestens einem Abschnitt der Kurve für Wärmestrom oder zeitliche Änderung der Temperatur, die für die Untersuchung der Probe 1 erhalten wurde, errechnet und mit Integralwerten von Referenzkurven verglichen werden, um die Auswertung noch zu verbessern.

Ferner sind Ausführungsbeispiele möglich, bei denen im Schritt S5 der erhaltene Verlauf des Wärmestroms oder der zeitlichen Änderung der Temperatur mit dem Referenzverlauf jeweils in deren Gesamtheit direkt verglichen wird. Hierbei werden vorzugsweise die Kurvenform und die Referenzkurvenbeschaffenheit unter Einsatz eines Mustererkennungsalgorithmus, beispielsweise eines lernfähigen Algorithmus, und/oder eines Verfahrens künstlicher Intelligenz, miteinander verglichen.

Bei einigen Ausführungsbeispielen kann zudem im Schritt S5 vorgesehen sein, bei Vorliegen eines Materialgemisches in der Probe 1 die Ergebniskurve, mit anderen Worten die für den Wärmestrom oder die zeitliche Änderung der Temperatur, jeweils aufgetragen über der Zeit oder der Temperatur, erhaltene Kurve, mit mehreren Referenzkurven "anzufitten", d.h. ein Fitting mehrerer Referenzkurven an das eigentliche Messergebnis durchzuführen, und aus den sich ergebenden Gewichten der einzelnen Referenzkurven auf die Zusammensetzung des Probematerials der Probe 1 zu schließen. Für ein derartiges Fitting kann beispielsweise ein charakteristischer Peak jeder der Kurven in Frage kommen, wobei das Fitting dann beispielsweise auf einen Kurvenabschnitt mit einem solchen Peak fokussiert wird.

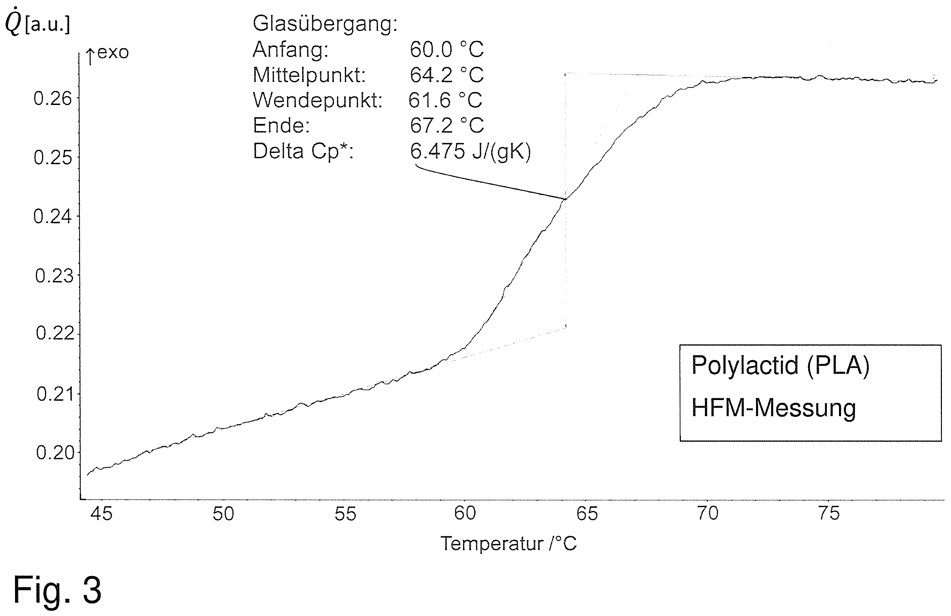

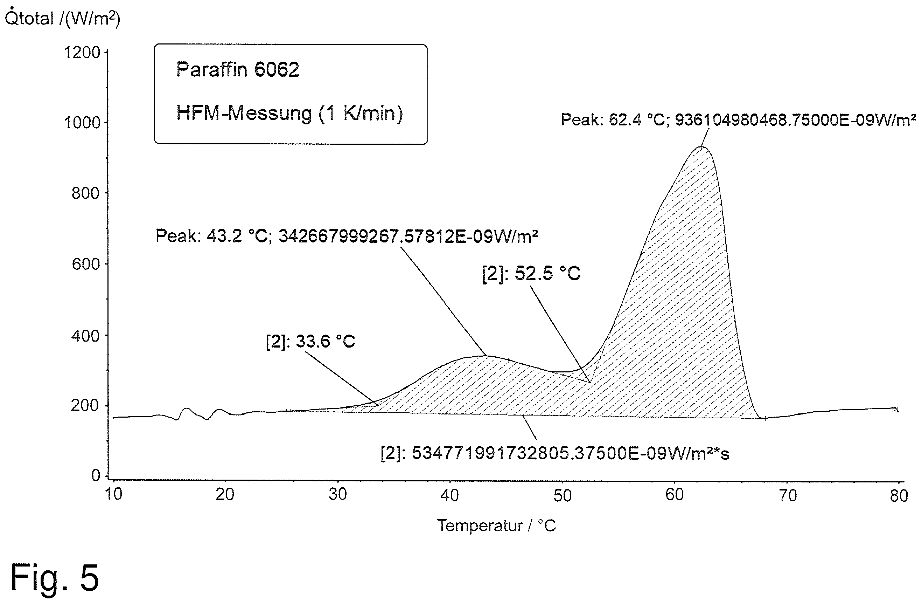

Wärmestromkurven, die für eine eingeprägte Temperaturänderung in Gestalt einer linearen Rampe mit der HFM-ähnlichen Anordnung 100 gemäß Fig. 1 gemäß Ausführungsbeispielen der Erfindung erhalten wurden und daher als "HFM-Messung" bezeichnet sind, sind in Fig. 3 für ein Polylactid (PLA) und in Fig. 5 für ein Paraffin, hier beispielhaft Paraffin 6062, dargestellt. Einige charakteristische, die Kurvenform im jeweils dargestellten Abschnitt kennzeichnende Werte, die aus der *Q̇*(*T*)-Kurve jeweils abgeleitet wurden, sind in den Fig. 3, 5 angegeben.

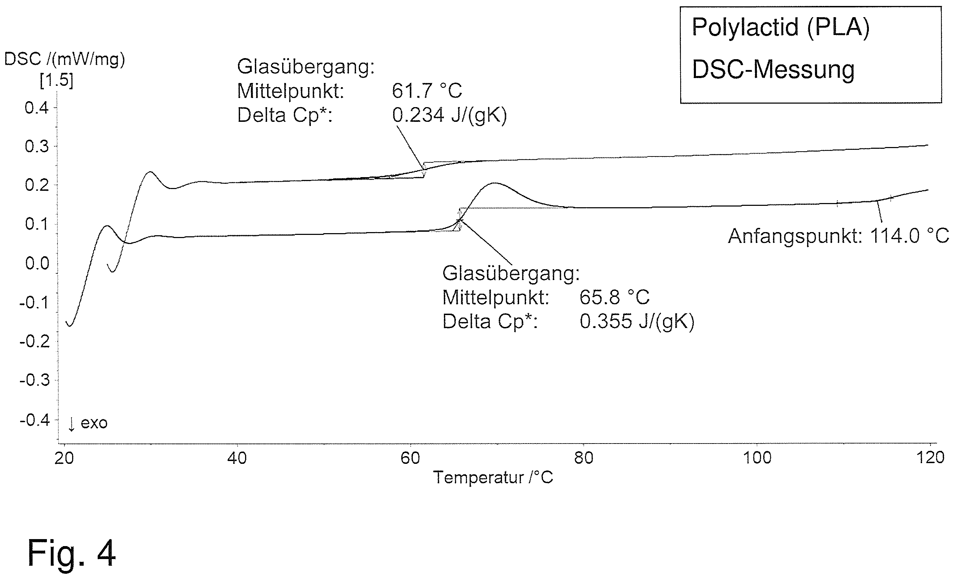

Es illustriert Fig. 3 beispielsweise, dass sich bei der "HFM-Messung" ein Glasübergang an PLA in der zweiten Aufheizung als eine Stufe bei ca. 65 Grad Celsius, mit einem Mittelpunkt der Stufe bei 64,2 °C, zeigt. In Fig. 3 ist der Wärmestrom *Q̇* zur Illustration insbesondere der Kurvenform und deren Charakterisierung in einer willkürlichen Einheit (arbitrary unit, mit a.u. abgekürzt) aufgetragen. Zum Vergleich zeigt Fig. 4 das Ergebnis einer konventionellen DSC-Messung (dynamische Differenzkalorimetrie) für zwei Aufheizungen des gleichen Polylactids, allerdings an einer erheblich geringeren Probenmasse. Bei dem in Fig. 4 gezeigten Beispiel wird ein Mittelpunkt der dem Glasübergang korrespondierenden Stufe von 65,8 °C bei der ersten Aufheizung und 61,7 °C bei der zweiten Aufheizung, jeweils im konventionellen DSC-Verfahren, erhalten.

Der Vergleich des gemäß einem Ausführungsbeispiel der Erfindung erhaltenen Ergebnisses in Fig. 3 für die zweite Aufheizung mit den DSC-Ergebnissen in Fig. 4 für die erste und zweite Aufheizung zeigt generell eine gute Ähnlichkeit hinsichtlich der Kurvenform und der Zahlenwerte. Das von der Erfindung vorgeschlagene Verfahren macht es somit möglich, Resultate nach Art einer DSC zu erzielen. Referenzkurven für bekannte Probematerialien zum Aufbau einer Datenbank, wie weiter oben beschrieben, für die Identifikation und/oder Klassifizierung der Komponenten eines zu untersuchenden Probematerials werden vorzugsweise im HFM-ähnlichen Verfahren der Erfindung, etwa mittels der Anordnung 100, erzeugt, um eine möglichst gute Vergleichbarkeit zu erreichen.

Vielfach kann es vorteilhaft sein, in den Verfahren gemäß Ausführungsbeispielen der Erfindung die zweite Aufheizung des Probematerials, wie etwa für PLA in Fig. 3, heranzuziehen, da die zweite Aufheizung von einem besser definierten Ausgangszustand ausgeht.

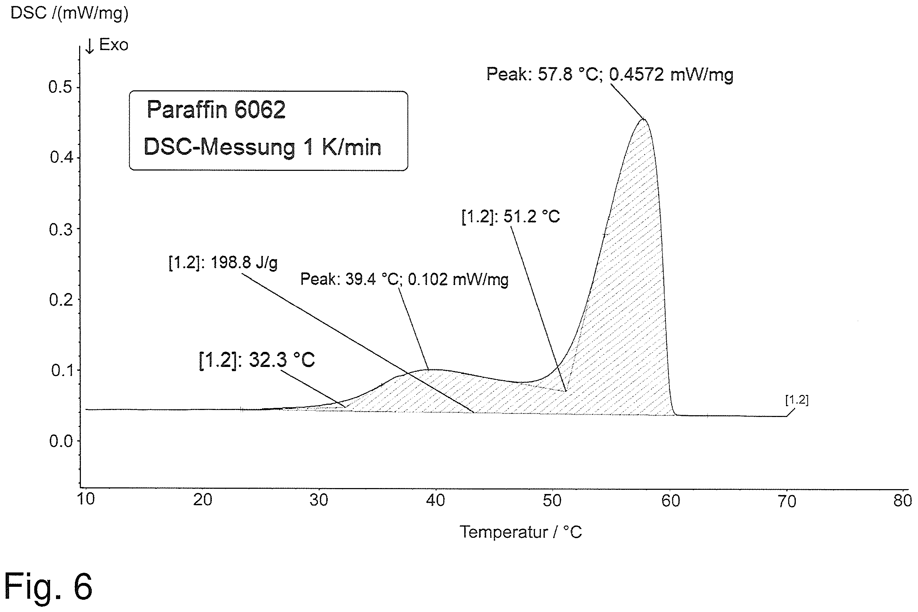

Für die beispielhaft gezeigte, mit einem Verfahren gemäß einem Ausführungsbeispiel durchgeführte Auswertung des Wärmestromverlaufs, der bei eingeprägter kontinuierlicher Temperaturänderung in Form einer linearen Rampe mit einer Steigung von 1 Kelvin pro Minute mittels einer Anordnung 100 gemäß Fig. 1 für das Paraffin 6062 bei erster Aufheizung gemessen wurde, ist in Fig. 5 zusätzlich zu den Abszissen- und Ordinatenwerten zweier Peaks und weiteren die Kurvenform charakterisierenden Temperaturwerten ein Integralwert unter einem Abschnitt der erhaltenen Kurve schraffiert dargestellt. Fig. 5 zeigt einen Schmelzübergang des Paraffins 6062. Erwähnt sei, dass im Falle des Paraffins 6062 die zweite Aufheizung sehr ähnliche Ergebnisse liefert. Zum Vergleich ist in Fig. 6 wiederum das Ergebnis einer konventionellen DSC-Messung für die erste Aufheizung des gleichen Paraffins dargestellt, was wiederum die gute Übereinstimmung mit dem gemäß einem Ausführungsbeispiel der Erfindung erhaltenen Ergebnis in Fig. 5, insbesondere auch hinsichtlich der Kurvenform, aufzeigt.

Werden beispielsweise die unterschiedlichen Paraffine 6062 und 5254 gemäß einem noch weiteren Ausführungsbeispiel untersucht und das Messergebnis ausgewertet, so zeigt sich deren Schmelzen als Auftreten von Maxima im einem Temperaturbereich zwischen 50 °C und 70 °C.

Das Verfahren, wie es vorstehend anhand von Ausführungsbeispielen beschrieben ist, kann Anwendung zum Beispiel in der Reinheitsbestimmung eines Kunststoffrecyclats, beispielsweise eines aus recycliertem Kunststoff hergestellten Granulats, finden. Hierbei bietet das Verfahren gemäß den vorstehend erläuterten Ausführungsbeispielen eine einfache, zeit- und kostensparende sowie genaue und zuverlässige Möglichkeit, zu bestimmen, ob das Granulat durch den Recyclingprozess Beimischungen oder Verunreinigungen, insbesondere durch andere Kunststoffe, enthält und in welchem Ausmaß. Die vorstehenden Ausführungsbeispiele, die in symmetrischer Weise Wärme in die Probe 1 mit der platten- oder scheibenartigen Geometrie 2 durch Hauptoberflächen 21, 22 der letzteren einbringen bzw. aus dieser abführen, ermöglicht durch die relativ großen möglichen Einwaagen an Probematerial die Untersuchung von Chargen im industriell relevanten Maßstab, wie bei Recyclingprozessen. Der Aufwand für die Untersuchung der Charge ist hierbei überschaubar und praktikabel.

Die Untersuchung der Charge durch Untersuchung der Probe 1 kann beispielsweise in dem das Granulat herstellenden Betrieb zur Qualitätskontrolle der ausgehenden Charge oder analog beim Kunden, etwa einem Kunststoffverarbeiter, zur Eingangskontrolle einfach, schnell und effizient genutzt werden und kann zuverlässige, genaue Ergebnisse erbringen. Das Probematerial der Probe 1 kann ein bereits compoundierter Kunststoff sein.

Von Vorteil ist bei der Erfindung, dass eine Messung an einem Referenzmaterial oder eine Leermessung als Referenz, zeitgleich mit den Messungen am eigentlichen Untersuchungsobjekt, also der Probe 1, wie dies etwa bei dem herkömmlichen DSC-Verfahren typischerweise mittels eines zweiten, leeren Tiegels vorgesehen ist, nicht notwendig ist. Die Anordnung 100 der Fig. 1, wie sie für die hier beschriebenen Ausführungsbeispiele verwendet wird, beinhaltet keine Einrichtungen zur gleichzeitigen Durchführung einer Referenzmessung. Auch ein leerer "Zwilling" der Anordnung 100 als Referenz wird zur Durchführung der Verfahren gemäß den hier beschriebenen Ausführungsbeispielen der Erfindung nicht benötigt. Der Aufwand für die Durchführung der Verfahren gemäß den hier beschriebenen Ausführungsbeispielen ist somit sowohl in apparativer Hinsicht als auch hinsichtlich der Handhabung vorteilhaft begrenzt.

Je nach Anwendungsfall und den zu untersuchenden Probematerialien kann die Anordnung 100 analog einer als solcher grundsätzlich bekannten HFM-Apparatur, wie etwa der in ATSM C1784 beschriebenen, ausgebildet sein und entsprechend den Temperaturen, bei denen die zu beobachtenden exo-/endothermen Vorgänge im Probematerial erwartet werden, modifiziert sein. Hierbei kann eine HFM-Apparatur beispielsweise zur Durchführung des in den vorstehenden Ausführungsbeispielen beschriebenen Verfahrens für höhere und/oder niedrigere Temperaturen und/oder einen breiteren Temperaturbereich, den die Apparatur ermöglichen und für den sie geeignet sein soll, modifiziert sein, beispielsweise hinsichtlich der Leistung, Auslegung oder Dimensionierung der Heiz- und Kühleinrichtungen 7, 8, 9, 10, eventueller Dichtmaterialien und ggf weiterer Bestandteile. Für die Anwendung im Bereich des Kunststoffrecyclings wird die Anordnung 100 somit beispielhaft derart ausgebildet, dass Phasen- und/oder Glasübergänge der relevanten, erwartbaren Kunststoffe als Haupt- und Verunreinigungsbestandteile des Probematerials von dem Temperaturbereich, den die Anordnung 100 ermöglicht, zuverlässig erfasst werden.

Wenngleich die Erfindung vorstehend anhand bevorzugter Ausführungsbeispiele vollständig beschrieben wurde, ist sie nicht darauf beschränkt, sondern auf vielfältige Art und Weise modifizierbar.

### Liste der Referenzzeichen

- 1: Probe
- 2: platten- oder scheibenartige Geometrie
- 3, 4: temperierbares plattenförmiges Element
- 5, 6: Wärmestromsensor
- 7, 8: Temperiersystem
- 9, 10: Kühlkörper
- 11: erste Seite
- 12: zweite Seite
- 21: erste Hauptoberfläche
- 22: zweite Hauptoberfläche
- 30: Verfahreinrichtung
- 40: Dickenmesseinrichtung
- 50: Kühlsystem
- 60: Datenverarbeitungs- und Steuerungssystem
- 70: Umhüllung
- 80: Dichtelement
- 100: Anordnung
- D: Dickenrichtung
- F: Last
- Qa: Wärmeabfluss
- Qz: Wärmezufluss
- S1, S2: Schritt
- S3a, S3b: Schritt
- S4a, S4b: Schritt
- S5, S6: Schritt
- t: Dicke
- x, y: Koordinatenrichtung

## Patentansprüche

1. Verfahren zum Identifizieren mindestens einer Komponente eines Probematerials, mit dem eine Probe (1) gebildet ist, umfassend:
Bereitstellen der Probe (1), wobei das Probematerial mit einer platten- oder scheibenartigen Geometrie (2) ausgebildet wird oder das Probematerial in einem Raumgebiet mit einer platten- oder scheibenartigen Geometrie (2) angeordnet wird;
Anordnen der Probe (1) zwischen zwei temperierbaren plattenförmigen Elementen (3, 4) derart, dass sich die temperierbaren plattenförmigen Elemente (3, 4) entlang jeweils einer von zwei entgegengesetzten Hauptoberflächen (21, 22) der platten- oder scheibenartigen Geometrie (2) erstrecken;
Herbeiführen einer Temperaturänderung der Probe (1) durch Herbeiführen eines Wärmezuflusses (Qz) in die Probe (1) oder eines Wärmeabflusses (Qa) aus der Probe (1) in einer symmetrischen Weise durch die beiden Hauptoberflächen (21, 22) der platten- oder scheibenartigen Geometrie (2), wobei die Temperaturänderung mittels Temperieren der plattenförmigen Elemente (3, 4) bewirkt wird und einen Temperaturbereich bestreicht, innerhalb dessen mindestens eine endotherme oder exotherme Veränderung der mindestens einen Komponente des Probematerials auftritt;
Erfassen eines in die Probe (1) hinein oder aus der Probe (1) heraus fließenden Wärmestroms und hieraus Erhalten eines als Kurve darstellbaren Verlaufs des Wärmestroms in Abhängigkeit von einer Temperatur der Probe (1) oder der temperierbaren plattenförmigen Elemente (3, 4) oder in Abhängigkeit von der Zeit, oder
Erfassen einer Temperatur der Probe (1) oder der temperierbaren plattenförmigen Elemente (3, 4) und hieraus Erhalten eines als Kurve darstellbaren Verlaufs einer zeitlichen Änderung der Temperatur in Abhängigkeit von der Temperatur oder in Abhängigkeit von der Zeit;
Vergleichen einer Kurvenbeschaffenheit, insbesondere einschließlich einer Kurvenform, des erhaltenen Verlaufs des Wärmestroms oder der zeitlichen Änderung der Temperatur über der Temperatur oder der Zeit jeweils mit einer Referenzkurvenbeschaffenheit eines oder mehrerer Referenzverläufe; und
Identifizieren der mindestens einen Komponente des Probematerials basierend auf dem Ergebnis des Vergleichs.

2. Verfahren nach Anspruch 1, **dadurch gekennzeichnet, dass** bei dem Herbeiführen der Temperaturänderung der Probe (1) das Probematerial einem kontinuierlichen Temperaturverlauf, insbesondere einer vordefinierten Temperaturrampe, vorzugsweise einer linear mit der Zeit ansteigenden oder abfallenden Temperaturrampe, unterworfen wird.

3. Verfahren nach Anspruch 1, **dadurch gekennzeichnet, dass** das Herbeiführen der Temperaturänderung der Probe (1) in der Weise erfolgt, dass ein über der Zeit konstanter Wärmestrom der Probe (1) zugeführt oder aus der Probe (1) abgeführt wird.

4. Verfahren nach einem der vorstehenden Ansprüche, **dadurch gekennzeichnet, dass** innerhalb des bei der Temperaturänderung bestrichenen Temperaturbereichs die mindestens eine Komponente des Probematerials einen Phasenübergang und/oder einen Glasübergang durchläuft.

5. Verfahren nach einem der vorstehenden Ansprüche, **dadurch gekennzeichnet, dass** das Probematerial während der Temperaturänderung ganz oder teilweise aufgeschmolzen oder eingefroren wird.

6. Verfahren nach einem der vorstehenden Ansprüche, **dadurch gekennzeichnet, dass** das Probematerial mittels Temperierens der temperierbaren plattenförmigen Elemente (3, 4) zwei oder mehr Temperaturänderungsvorgängen unterzogen wird, insbesondere einem oder mehreren Heizvorgängen und einem oder mehreren Kühlvorgängen.

7. Verfahren nach einem der vorstehenden Ansprüche, **dadurch gekennzeichnet, dass** ein jeweils von dem temperierbaren plattenförmigen Element (3, 4) in die Probe (1) oder umgekehrt fließender Wärmestrom jeweils zwischen dem temperierbaren plattenförmigen Element (3, 4) und der dem temperierbaren plattenförmigen Element (3, 4) zugewandten Seite (11, 12) der Probe (1) im Bereich der Hauptoberfläche (21, 22) der platten- oder scheibenartigen Geometrie (2), der das temperierbare plattenförmige Element (3, 4) benachbart ist, erfasst wird, insbesondere jeweils mittels eines Wärmestromsensors (5, 6), beispielsweise eines flächig ausgebildeten Wärmestromsensors (5, 6).

8. Verfahren nach Anspruch 7, **dadurch gekennzeichnet, dass** die Wärmestromsensoren (5, 6) jeweils flächig in einem Mittenbereich einer der Hauptoberflächen (21, 22) der platten- oder scheibenartigen Geometrie (2) zwischen dem dieser Hauptoberfläche (21, 22) benachbarten temperierbaren plattenförmigen Element (3, 4) und der Probe (1) angeordnet werden.

9. Verfahren nach einem der vorstehenden Ansprüche, **dadurch gekennzeichnet, dass** eines oder beide der temperierbaren plattenförmigen Elemente (3, 4) vor dem Herbeiführen der Temperaturänderung der Probe (1) auf die Probe (1) zu bewegt wird/werden, wobei vermittelt über die temperierbaren plattenförmigen Elemente (3, 4) eine vordefinierte Kraft auf die Probe (1) in einer Dickenrichtung (D) der platten- oder scheibenartigen Geometrie (2) aufgebracht oder eine vordefinierte Dicke (t) der Probe (1) in der Dickenrichtung (D) der platten- oder scheibenartigen Geometrie (2) eingestellt wird.

10. Verfahren nach einem der vorstehenden Ansprüche, **dadurch gekennzeichnet, dass** die Temperatur der Probe (1) an einer Oberfläche der Probe oder in einem Inneren der Probe (1) erfasst wird und/oder dass die Temperatur des temperierbaren plattenförmigen Elements (3, 4) jeweils an einer Oberfläche desselben oder in einem Inneren desselben erfasst wird.

11. Verfahren nach einem der vorstehenden Ansprüche, **dadurch gekennzeichnet, dass** das Vergleichen der Kurvenbeschaffenheit und der Referenzkurvenbeschaffenheit unter Verwendung korrespondierender, die Kurvenform und die Referenzkurvenform jeweils zumindest abschnittsweise charakterisierender Abszissen- und/oder Ordinatenwerte durchgeführt wird.

12. Verfahren nach einem der vorstehenden Ansprüche, **dadurch gekennzeichnet, dass** das Vergleichen der Kurvenbeschaffenheit und der Referenzkurvenbeschaffenheit anhand mindestens einer charakteristischen Stufe und/oder mindestens eines charakteristischen Extremums, insbesondere Peaks, jeweils des erhaltenen Verlaufs des Wärmestroms oder der zeitlichen Änderung der Temperatur sowie des Referenzverlaufs durchgeführt wird, wobei die Stufe oder das Extremum jeweils insbesondere einer Umwandlung innerhalb des Probematerials korrespondiert.

13. Verfahren nach einem der vorstehenden Ansprüche, **dadurch gekennzeichnet, dass** für mindestens eine Stufe oder mindestens ein Extremum, insbesondere einen Peak, des erhaltenen Verlaufs des Wärmestroms oder der zeitlichen Änderung der Temperatur mindestens ein Abszissenwert, der einem der Stufe oder dem Extremum innerhalb der Kurvenform zugeordneten Anfangspunkt oder Endpunkt oder Mittelpunkt oder Wendepunkt oder Extremalwert korrespondiert, ermittelt und mit mindestens einem korrespondierenden Abszissenwert mindestens eines Referenzverlaufs verglichen wird.

14. Verfahren nach einem der vorstehenden Ansprüche, **dadurch gekennzeichnet, dass** für mindestens eine Stufe des erhaltenen Verlaufs des Wärmestroms oder der zeitlichen Änderung der Temperatur eine Höhe der Stufe ermittelt und mit einer Höhe mindestens einer Stufe mindestens eines Referenzverlaufs verglichen wird, und/oder dass für mindestens ein Extremum, insbesondere einen Peak, des erhaltenen Verlaufs des Wärmestroms oder der zeitlichen Änderung der Temperatur der zugeordnete Extremalwert des Wärmestroms oder der zeitlichen Änderung der Temperatur ermittelt und mit einem Extremalwert mindestens eines Extremums, insbesondere Peaks, mindestens eines Referenzverlaufs verglichen wird.

15. Verfahren nach einem der vorstehenden Ansprüche, **dadurch gekennzeichnet, dass** mindestens ein charakteristischer Integralwert, insbesondere eine Fläche unter mindestens einem Abschnitt des als Kurve dargestellten, erhaltenen Verlaufs des Wärmestroms oder der zeitlichen Änderung der Temperatur, beispielsweise unter einem Peak, ermittelt und mit einem charakteristischen Integralwert des Referenzverlaufs verglichen wird und dass das Identifizieren der mindestens einen Komponente des Probematerials zusätzlich auf dem Ergebnis des Vergleichs der charakteristischen Integralwerte basierend durchgeführt wird.

16. Verfahren nach einem der vorstehenden Ansprüche, **dadurch gekennzeichnet, dass** der erhaltene Verlauf des Wärmestroms oder der zeitlichen Änderung der Temperatur mit dem Referenzverlauf jeweils in deren Gesamtheit direkt verglichen wird.

17. Verfahren nach einem der vorstehenden Ansprüche, **dadurch gekennzeichnet, dass** das Vergleichen der Kurvenbeschaffenheit, insbesondere Kurvenform, und der Referenzkurvenbeschaffenheit, insbesondere Referenzkurvenform, den Einsatz eines Mustererkennungsalgorithmus, beispielsweise eines lernfähigen Algorithmus, und/oder eines Verfahrens künstlicher Intelligenz umfasst.

18. Verfahren nach einem der vorstehenden Ansprüche, **dadurch gekennzeichnet, dass** als Referenzverläufe eine Vielzahl jeweils für ein bekanntes Probematerial oder Probematerialgemisch vorab ermittelter Verläufe des Wärmestroms oder der zeitlichen Änderung der Temperatur verwendet werden.

19. Verfahren nach einem der vorstehenden Ansprüche, **dadurch gekennzeichnet, dass** die Referenzverläufe in Form einer Datensammlung bereitgestellt werden, welche Referenzverläufe einer anwendungsspezifischen Auswahl an Reinstoffen und Gemischen, beispielsweise Kunststoffen und Kunststoffgemischen, beinhaltet.

20. Verfahren nach einem der vorstehenden Ansprüche, **dadurch gekennzeichnet, dass** das Probematerial als ein loses Material mit einer Vielzahl einzelner Stücke bereitgestellt wird, beispielsweise als ein Granulat oder ein Pulver, oder dass das Probematerial als ein zusammenhängender Körper bereitgestellt wird.

21. Verfahren nach einem der vorstehenden Ansprüche, **dadurch gekennzeichnet, dass** die Erfassung des Verlaufs des Wärmestroms oder der Temperatur in Abwesenheit einer gleichzeitigen Referenzmessung, insbesondere an einer geometrisch mit der Probe (1) übereinstimmenden, mit einem vorab bekannten Material gebildeten Referenz oder durch Leermessung als Referenz, innerhalb derselben Untersuchungsanordnung (100) oder mittels einer weiteren identischen Untersuchungsanordnung (100) erfolgt.

22. Verfahren nach einem der vorstehenden Ansprüche, **dadurch gekennzeichnet, dass** die untersuchte Probe (1) eine Masse von mehr als etwa 10 Gramm, beispielsweise zwischen etwa 10 Gramm und etwa 10 Kilogramm, bevorzugt zwischen etwa 10 Gramm und etwa 1 Kilogramm, weiter bevorzugt zwischen etwa 10 Gramm und etwa 200 Gramm, beispielsweise zwischen etwa 10 Gramm und etwa 100 Gramm, aufweist.

23. Verfahren nach einem der vorstehenden Ansprüche, **dadurch gekennzeichnet, dass** das Probematerial ein Gemisch ist und mittels des Verfahrens ein Material oder mehrere Materialien, das/die in der Probe (1) enthalten ist/sind, identifiziert wird/werden.

24. Verfahren nach einem der vorstehenden Ansprüche, **dadurch gekennzeichnet, dass** das Verfahren zur Reinheitsbestimmung eines Kunststoffrecyclats verwendet wird, insbesondere zur Identifizierung eventueller Beimischungen oder Verunreinigungen und/oder zur Bestimmung eines Ausmaßes dieser.

25. Verfahren nach einem der vorstehenden Ansprüche, **dadurch gekennzeichnet, dass** das Verfahren in einer Prozesskette beim Kunststoff-Recycling verwendet wird.

26. Verfahren nach einem der vorstehenden Ansprüche, **dadurch gekennzeichnet, dass** als das Probematerial der Probe (1) ein compoundiertes Stoffgemisch, insbesondere ein compoundierter Kunststoff, bereitgestellt wird.

## Claims

1. A method for identifying at least one component of a sample material, with which a sample (1) is formed, comprising:
providing the sample (1), wherein the sample material is formed with a plate-like or disk-like geometry (2) or the sample material is arranged in a spatial region with a plate-like or disk-like geometry (2);
arranging the sample (1) between two temperature-controllable plate-shaped elements (3, 4) in such a way that the temperature-controllable plate-shaped elements (3, 4) extend along a respective one of two opposite main surfaces (21, 22) of the plate-like or disk-like geometry (2);
bringing about a temperature change of the sample (1) by bringing about a heat inflow (Qz) into the sample (1) or a heat outflow (Qa) out of the sample (1) in a symmetrical manner through the two main surfaces (21, 22) of the plate-like or disk-like geometry (2), wherein the temperature change is effected by controlling the temperature of the plate-shaped elements (3, 4) and covers a temperature range, within which at least one endothermic or exothermic change of the at least one component of the sample material occurs;
detecting a heat flow flowing into the sample (1) or out of the sample (1) and obtaining from this a profile of the heat flow, which can be represented as curve, as a function of a temperature of the sample (1) or of the temperature-controllable plate-shaped elements (3, 4) or as a function of time, or
detecting a temperature of the sample (1) or of the temperature-controllable plate-shaped elements (3, 4), and obtaining from this a profile of a temporal change of the temperature, which can be represented as curve, as a function of the temperature or as a function of time;
comparing a curve state, in particular including a curve shape, of the obtained profile of the heat flow or of the temporal change of the temperature over temperature or time in each case to a reference curve state of one or several reference profiles; and
identifying the at least one component of the sample material based on the result of the comparison.

2. The method according to claim 1, **characterized in that** the sample material is subjected to a continuous temperature profile, in particular a predefined temperature ramp, preferably a temperature ramp, which rises or falls linearly over time, when bringing about the temperature change of the sample (1).

3. The method according to claim 1, **characterized in that** the bringing about of the temperature change of the sample (1) takes place in such a way that a heat flow, which is constant over time, is supplied to the sample (1) or is removed from the sample (1).

4. The method according to one of the preceding claims, **characterized in that** the at least one component of the sample material runs through a phase transition and/or a glass transition within the temperature range covered during the temperature change.

5. The method according to one of the preceding claims, **characterized in that** the sample material is melted or frozen completely or partly during the temperature change.

6. The method according to one of the preceding claims, **characterized in that** the sample material is subjected to two or more temperature change processes, in particular to one or several heating processes and one or several cooling processes, by means of controlling the temperature of the temperature-controllable plate-shaped elements (3, 4).

7. The method according to one of the preceding claims, **characterized in that** a heat flow, which in each case flows from the temperature-controllable plate-shaped element (3, 4) into the sample (1) or vice versa, is in each case detected between the temperature-controllable plate-shaped element (3, 4) and the side (11, 12) of the sample (1) facing the temperature-controllable plate-shaped element (3, 4) in the region of the main surface (21, 22) of the plate-like or disk-like geometry (2), to which the temperature-controllable plate-shaped element (3, 4) is adjacent, in particular in each case by means of a heat flux transducer (5, 6), for example of a heat flux transducer (5, 6), which is formed in a planar manner.

8. The method according to claim 7, **characterized in that** the heat flux transducers (5, 6) are in each case arranged in a planar manner in a central region of one of the main surfaces (21, 22) of the plate-like or disk-like geometry (2) between the temperature-controllable plate-shaped element (3, 4) adjacent to this main surface (21, 22) and the sample (1).

9. The method according to one of the preceding claims, **characterized in that** one or both of the temperature-controllable plate-shaped elements (3, 4) is/are moved towards the sample (1) prior to bringing about the temperature change of the sample (1), wherein, conveyed via the temperature-controllable plate-shaped elements (3, 4), a predefined force is applied to the sample (1) in a thickness direction (D) of the plate-like or disk-like geometry (2) or a predefined thickness (t) of the sample (1) is adjusted in the thickness direction (D) of the plate-like or disk-like geometry (2).

10. The method according to one of the preceding claims, **characterized in that** the temperature of the sample (1) is detected on a surface of the sample or in an interior of the sample (1) and/or that the temperature of the temperature-controllable plate-shaped element (3, 4) is in each case detected on a surface thereof or in an interior thereof.

11. The method according to one of the preceding claims, **characterized in that** the comparing of the curve state and of the reference curve state is carried out by using corresponding abscissa and/or ordinate values, which in each case characterize the curve shape and the reference curve shape at least in sections.

12. The method according to one of the preceding claims, **characterized in that** the comparing of the curve state and of the reference curve state is carried out by means of at least one characteristic level and/or of at least one characteristic extremum, in particular peak, in each case of the obtained profile of the heat flow or of the temporal change of the temperature as well as of the reference profile, wherein the level or the extremum in each case corresponds in particular to a conversion within the sample material.

13. The method according to one of the preceding claims, **characterized in that** at least one abscissa value, which corresponds to an onset point or endpoint or midpoint or turning point or extreme value assigned to the level or the extremum within the curve shape, is determined for at least one level or at least one extremum, in particular a peak, of the obtained profile of the heat flow or the temporal change of the temperature, and is compared to at least one corresponding abscissa value of at least one reference profile.

14. The method according to one of the preceding claims, **characterized in that** a height of the level is determined for at least one level of the obtained profile of the heat flow or of the temporal change of the temperature, and is compared to at least one height of at least one level of at least one reference profile, and/or that the assigned extreme value of the heat flow or of the temporal change of the temperature is determined for at least one extremum, in particular a peak, of the obtained profile of the heat flow or of the temporal change of the temperature, and is compared to an extreme value of at least one extremum, in particular peak, of at least one refence profile.

15. The method according to one of the preceding claims, **characterized in that** at least one characteristic integral value, in particular a surface below at least one section of the obtained profile of the heat flow represented as curve or of the temporal change of the temperature, for example under a peak, is determined and compared to a characteristic integral value of the reference profile and that the identification of the at least one component of the sample material is additionally carried out based on the result of the comparison of the characteristic integral values.

16. The method according to one of the preceding claims, **characterized in that** the obtained profile of the heat flow or of the temporal change of the temperature is compared directly to the reference profile in each case in the totality thereof.

17. The method according to one of the preceding claims, **characterized in that** the comparing of the curve state, in particular curve shape, and of the reference curve state, in particular reference curve shape, includes the use of a pattern recognition algorithm, for example of an adaptive algorithm and/or of an artificial intelligence method.

18. The method according to one of the preceding claims, **characterized in that** a plurality of profiles of the heat flow or of the temporal change of the temperature, which are in each case determined beforehand for a known sample material or sample material mixture, are used as reference profiles.

19. The method according to one of the preceding claims, **characterized in that** the reference profiles are provided in the form of a data collection, which includes reference profiles of an application-specific selection of pure substances and mixtures, for example plastics and plastic mixtures.

20. The method according to one of the preceding claims, **characterized in that** the sample material is provided as a loose material with a plurality of individual pieces, for example as a granulate or a powder or that the sample material is provided as a cohesive body.

21. The method according to one of the preceding claims, **characterized in that** the detection of the profile of the heat flow or of the temperature takes place in the absence of a simultaneous reference measurement, in particular on a reference, which geometrically corresponds to the sample (1) and which is formed with a material known beforehand, or by means of empty measurement as reference, within the same analysis arrangement (100) or by means of a further identical analysis arrangement (100).

22. The method according to one of the preceding claims, **characterized in that** the analyzed sample (1) has a mass of more than approximately 10 grams, for example between approximately 10 grams and approximately 10 kilograms, preferably between approximately 10 grams and approximately 1 kilogram, more preferably between approximately 10 grams and approximately 200 grams, for example between approximately 10 grams and approximately 100 grams.

23. The method according to one of the preceding claims, **characterized in that** the sample material is a mixture and that a material or several materials, which is/are contained in the sample (1), is/are identified by means of the method.

24. The method according to one of the preceding claims, **characterized in that** the method is used for determining the purity of a plastics recyclate, in particular for identifying possible admixtures or contaminations and/or for determining an extent thereof.

25. The method according to one of the preceding claims, **characterized in that** the method is used in a process chain during the plastics recycling.

26. The method according to one of the preceding claims, **characterized in that** a compounded substance mixture, in particular a compounded plastic, is provided as the sample material of the sample (1).

## Revendications

1. Procédé, destiné à identifier au moins un composant d'une matière échantillon, en laquelle est constitué un échantillon (1), comprenant :
la mise à disposition de l'échantillon (1), la matière échantillon étant conçue avec une géométrie (2) de type plaque ou disque ou la matière échantillon étant placée dans une zone d'espace pourvue d'une géométrie (2) de type plaque ou disque ;
le placement de l'échantillon (1) entre deux éléments (3, 4) tempérables en forme de plaques, de telle sorte que les éléments (3, 4) tempérables en forme de plaques s'étendent le long de chaque fois l'une de deux surfaces principales (21, 22) opposées de la géométrie (2) de type plaque ou disque ;
la provocation d'une variation de température de l'échantillon (1), en provoquant un afflux de chaleur (Qz) dans l'échantillon (1) ou une évacuation de température (Qa) hors de l'échantillon (1) de manière symétrique, à travers les deux surfaces principales (21, 22) de la géométrie (2) de type plaque ou disque, la variation de température étant induite en tempérant les éléments (3, 4) en forme de plaques et couvrant une plage de températures dans laquelle on assiste à une modification au moins endothermique ou exothermique de l'au moins un composant de la matière échantillon ;
la détection d'un flux thermique pénétrant dans l'échantillon (1) ou sortant de l'échantillon (1) et l'obtention à partir de celle-ci d'un trajet du flux thermique susceptible d'être représenté sous forme de courbe, en fonction d'une température de l'échantillon (1) ou des éléments (3, 4) tempérables en forme de plaques ou en fonction du temps, ou
la détection d'une température de l'échantillon (1) ou des éléments (3, 4) tempérables en forme de plaques et l'obtention à partir de celle-ci d'un trajet susceptible d'être représenté sous la forme d'une courbe d'une variation de la température dans le temps, en fonction de la température ou en fonction du temps ;
la comparaison d'une caractéristique de la courbe, incluant notamment une forme de la courbe du trajet obtenu du flux thermique ou de la variation de la température dans le temps sur la température ou sur le temps avec chaque respectivement une caractéristique d'une courbe de référence d'un ou de plusieurs trajets de référence ; et
l'identification de l'au moins un composant de la matière échantillon, sur la base du résultat de la comparaison.

2. Procédé selon la revendication 1, **caractérisé en ce que** lorsque l'on provoque la variation de température de l'échantillon (1), la matière échantillon est soumise à un trajet de température continu, notamment à une rampe de température prédéfinie, de préférence à une rampe de température linéaire, croissant ou décroissant dans le temps.

3. Procédé selon la revendication 1, **caractérisé en ce que** l'on provoque la variation de température de l'échantillon (1) de telle manière qu'un flux thermique constant dans le temps soit alimenté vers l'échantillon (1) ou évacué de l'échantillon (1).

4. Procédé selon l'une quelconque des revendications précédentes, **caractérisé en ce qu'**à l'intérieur de la plage de température couverte lors de la variation de température, l'au moins un composant de la matière échantillon traverse une transition de phase et / ou une transition vitreuse.

5. Procédé selon l'une quelconque des revendications précédentes, **caractérisé en ce que** pendant la variation de température, l'on fait fondre ou congeler totalement ou partiellement la matière échantillon.

6. Procédé selon l'une quelconque des revendications précédentes, **caractérisé en ce qu'**en tempérant les éléments (3, 4) tempérables en forme de plaques, l'on soumet la matière échantillon à deux processus de variation de température ou plus, notamment à un processus de chauffage ou plus et à un processus de refroidissement ou plus.

7. Procédé selon l'une quelconque des revendications précédentes, **caractérisé en ce que** l'on détecte un flux thermique circulant respectivement de l'élément (3, 4) tempérable en forme de plaque dans l'échantillon (1) ou inversement, respectivement entre l'élément (3, 4) tempérable en forme de plaque et le côté (11, 12) de l'échantillon (1) qui fait face à l'élément (3, 4) tempérable en forme de plaque, dans la zone de la surface principale (21, 22) de la géométrie (2) de type plaque ou disque qui est voisine de l'élément (3, 4) tempérable en forme de plaque, notamment respectivement au moyen d'un capteur de flux thermique (5, 6), par exemple d'un capteur de flux thermique (5, 6) de conception plane.

8. Procédé selon la revendication 7, **caractérisé en ce que** l'on place les capteurs de flux thermique (5, 6) respectivement à plat dans une zone centrale de l'une des surfaces principales (21, 22) de la géométrie (2) de type plaque ou disque, entre l'élément (3, 4) tempérable en forme de plaque voisin de ladite surface principale (21, 22) et l'échantillon (1).

9. Procédé selon l'une quelconque des revendications précédentes, **caractérisé en ce qu'**avant de provoquer la variation de température de l'échantillon (1), l'on déplace l'un ou les deux des éléments (3, 4) tempérables en forme de plaques vers l'échantillon (1), par l'intermédiaire des éléments (3, 4) tempérables en forme de plaques, une force prédéfinie étant exercée sur l'échantillon (1) dans une direction de l'épaisseur de la géométrie (2) de type plaque ou disque ou une épaisseur (t) prédéfinie de l'échantillon (1) étant réglée dans la direction de l'épaisseur de la géométrie (2) de type plaque ou disque.

10. Procédé selon l'une quelconque des revendications précédentes, **caractérisé en ce que** l'on détecte la température de l'échantillon (1) sur une surface de l'échantillon ou dans un intérieur de l'échantillon (1) et / ou **en ce que** l'on détecte la température de l'élément tempérable (3, 4) en forme de plaque, respectivement sur une surface de celui-ci ou dans un intérieur de celui-ci.

11. Procédé selon l'une quelconque des revendications précédentes, **caractérisé en ce que** l'on procède à la comparaison de la caractéristique de la courbe et de la caractéristique de la courbe de référence en utilisant des valeurs d'abscisses et / ou d'ordonnées correspondantes, caractérisant respectivement au moins par endroits la forme de la courbe et la forme de la courbe de référence.

12. Procédé selon l'une quelconque des revendications précédentes, **caractérisé en ce que** l'on procède à la comparaison de la caractéristique de la courbe et de la caractéristique de la courbe de référence à l'aide d'au moins un palier caractéristique et / ou d'au moins un extremum caractéristique, notamment de pics, respectivement du trajet obtenu du flux thermique ou de la variation de la température dans le temps, ainsi que du trajet de référence, le palier ou l'extremum correspondant respectivement notamment à une transformation à l'intérieur de la matière échantillon.

13. Procédé selon l'une quelconque des revendications précédentes, **caractérisé en ce que** pour au moins un palier ou au moins un extremum, notamment un pic du trajet obtenu du flux thermique ou de la variation de la température dans le temps, l'on détermine au moins une valeur d'abscisse qui correspond à un point de départ ou un point final ou à un point central ou à un point tournant ou à une valeur extrême affecté(e) au palier ou à l'extremum à l'intérieur de la forme de la courbe et on la compare avec au moins une valeur d'abscisse correspondante d'au moins un trajet de référence.

14. Procédé selon l'une quelconque des revendications précédentes, **caractérisé en ce que** pour au moins un palier du trajet obtenu du flux thermique ou de la variation de la température dans le temps, l'on détermine une hauteur du palier et on la compare avec une hauteur d'au moins un palier d'au moins un trajet de référence et / ou **en ce que** pour au moins un extremum, notamment un pic du trajet obtenu du flux thermique ou de la variation de la température dans le temps, l'on détermine la valeur extrême affectée du flux thermique ou de la variation dans le temps et on la compare avec une valeur extrême d'au moins un extremum, notamment d'un pic d'au moins un trajet de référence.

15. Procédé selon l'une quelconque des revendications précédentes, **caractérisé en ce que** l'on détermine au moins une valeur intégrale caractéristique, notamment une surface sous au moins un segment du trajet obtenu du flux thermique ou de la variation de la température dans le temps représenté sous la forme d'une courbe, par exemple sous un pic et on la compare avec une valeur intégrale caractéristique du trajet de référence et **en ce que** l'on procède à l'identification de l'au moins un composant de la matière échantillon, additionnellement sur la base du résultat de la comparaison des valeurs intégrales caractéristiques.

16. Procédé selon l'une quelconque des revendications précédentes, **caractérisé en ce que** l'on compare directement le trajet obtenu du flux thermique ou de la variation de la température dans le temps avec le trajet de référence respectivement dans sa totalité.

17. Procédé selon l'une quelconque des revendications précédentes, **caractérisé en ce que** la comparaison de la caractéristique de la courbe, notamment de la forme de la courbe et de la caractéristique de la courbe de référence, notamment de la forme de la courbe de référence comprend l'utilisation d'un algorithme de reconnaissance de modèles, par exemple d'un algorithme auto-apprenant et / ou d'un procédé d'intelligence artificielle.

18. Procédé selon l'une quelconque des revendications précédentes, **caractérisé en ce que** l'on utilise en tant que trajets de référence une pluralité de trajets du flux thermique ou de la variation de la température dans le temps préalablement déterminés respectivement pour une matière échantillon ou un mélange de matières échantillons connu(e).

19. Procédé selon l'une quelconque des revendications précédentes, **caractérisé en ce que** l'on met à disposition les trajets de référence sous la forme d'une collecte de données, laquelle contient des trajets de référence d'une sélection spécifique à l'application de substances pures et de mélanges, par exemple de matières plastiques et de mélanges de matières plastiques.

20. Procédé selon l'une quelconque des revendications précédentes, **caractérisé en ce que** l'on met à disposition la matière échantillon sous la forme d'une matière en vrac, présentant une pluralité de morceaux individuels, par exemple sous la forme de granulés ou d'une poudre ou **en ce que** l'on met à disposition la matière échantillon sous la forme d'un corps cohérent.

21. Procédé selon l'une quelconque des revendications précédentes, **caractérisé en ce que** la détection du trajet du flux thermique ou de la température s'effectue en l'absence d'une mesure de référence simultanée, notamment sur une référence concordant géométriquement avec l'échantillon (1), constituée d'une matière préalablement connue ou par une mesure à blanc servant de référence, dans la même configuration d'analyse (100) ou au moyen d'une configuration d'analyse (100) supplémentaire identique.

22. Procédé selon l'une quelconque des revendications précédentes, **caractérisé en ce que** l'échantillon (1) analysé présente une masse de plus d'environ 10 grammes, comprise par exemple entre environ 10 grammes et environ 10 kilogrammes, de manière préférentielle entre environ 10 grammes et environ 1 kilogramme, de manière plus préférentielle, entre environ 10 grammes et environ 200 grammes, par exemple entre environ 10 grammes et environ 100 grammes.

23. Procédé selon l'une quelconque des revendications précédentes, **caractérisé en ce que** la matière échantillon est un mélange et **en ce qu'**au moyen du procédé, l'on identifie une matière ou plusieurs matières qui est / sont contenue(s) dans l'échantillon (1).

24. Procédé selon l'une quelconque des revendications précédentes, **caractérisé en ce que** l'on utilise le procédé pour déterminer la pureté de matières plastiques recyclées, notamment pour identifier d'éventuel(le)s produits mélangés ou impuretés et / ou pour déterminer l'ampleur de ces dernier(ère)s.

25. Procédé selon l'une quelconque des revendications précédentes, **caractérisé en ce que** l'on utilise le procédé dans une chaîne de procédés, lors du recyclage de matières plastiques.

26. Procédé selon l'une quelconque des revendications précédentes, **caractérisé en ce que** l'on met à disposition en tant que matière échantillon de l'échantillon (1) un mélange de substances compoundé, notamment une matière plastique compoundée.
